# EUROPEAN PATENT APPLICATION

(11) **EP 3 848 397 A1**
(43) Date of publication of application: **14.07.2021**
(21) Application number: 19857108.5
(22) Date of filing: 03.09.2019
(51) Int. Cl.: C07K 19/00, C07K 16/28, C07K 14/715, C12N 15/62, C12N 15/63, C12N 5/10, A61K 39/395, A61K 38/21, A61P 35/00, A61P 31/12

(54) **FUSION PROTEIN AND ITS APPLICATION IN PREPARING MEDICINE FOR TREATING TUMOR AND/OR VIRAL INFECTION**

(30) Priority: 04.09.2018 CN 201811024783
(71) Applicant: NANJING UMAB-BIOPHARMA CO., LTD., Nanjing, Jiangsu 210042 (CN)
(72) Inventor: MO, Shifu, Nanjing, Jiangsu 210042 (CN); ZHAO, Yong, Nanjing, Jiangsu 210042 (CN); XU, Wei, Nanjing, Jiangsu 210042 (CN); WANG, Zhichao, Nanjing, Jiangsu 210042 (CN); YANG, Jie, Nanjing, Jiangsu 210042 (CN); GU, Liyun, Nanjing, Jiangsu 210042 (CN); DING, Dong, Nanjing, Jiangsu 210042 (CN)
(74) Representative: Potter Clarkson
(86) International application number: PCT/CN2019/104217
(87) International publication number: WO 2020/048454

(57) **Abstract**

The present invention provides a fusion protein and use thereof in preparing a medicament for treating tumors and/or viral infections, wherein the fusion protein comprises an antibody or antigen-binding fragment thereof that specifically binds to human OX40 and a human interferon.

## Description

### Technical Field

The present invention belongs to the field of biomedicine. Specifically, the present invention relates to a fusion protein and use thereof. More specifically, the present invention relates to a fusion protein comprising an anti-OX40 antibody and a human interferon, and use thereof in preparing a medicament for treating tumors and/or viral infections.

### Background Art

Human OX40 is mainly expressed on activated T cells, including CD4, CD8, Th, Treg cells, and etc. [1] The expression level of OX40 on naive T cells is low, but is up-regulated after antigen-induced stimulation and peaks within 12 h to 5-6 days. Similarly, the expression level of OX40L is also affected by the state of cell activation [1]. The expression of OX40L may be detected in APC cells 1-3 days after antigen stimulation. Interestingly, in addition to immune cells, muscle cells also express OX40L under the stimulation of inflammatory factors [2, 3], suggesting that OX40L-OX40 signaling pathway may play a wide role in the inflammatory response of the body.

The antigen-dependent activation of OX40L/OX40 costimulatory molecules is coupled to multiple signaling pathways in T cells. Crystal structure studies have shown that the binding of OX40L to OX40 may induce the trimerization of OX40-OX40L complex [4], thus forming a binding site for receptor-associated factor (TRAF) in the cell. The latter (TRAF2, 5) could then activate NF-κB signaling pathway and inhibit T cell apoptosis [2, 5, 6]. Studies have found that the activation of OX40 may result in the high expression of Bcl-2 and Bcl-xL [7], suggesting that OX40 may induce the expression of anti-apoptotic protein through NF-κB signaling pathway to achieve its function of inhibiting T cell apoptosis.

PKB/PI3K is another important signaling pathway located downstream of OX40. Studies have found that, on the one hand, the costimulatory signal of OX40 on T cells is necessary to maintain PKB activation, and on the other hand, constitutively activated PKB could antagonize the down-regulation of anti-apoptotic protein in T cells caused by OX40 deficiency. OX40 costimulatory signal can maintain the expression of Survivin through PKB/PI3K signaling pathway [8].

Finally, the activation of TCR and OX40 on T cells may also synergistically cause calcium flux and the activation of NFAT signaling pathway, regulating the expression of cytokines including IL-2, IL-4, IL-5, and IFN-γ [9].

In summary, the above studies indicate that activation of OX40 may regulate T cell proliferation and apoptosis, and cytokine secretion activity through NF-κB signaling pathway, PKB/PI3K signaling pathway and NFAT signaling pathway, thereby achieving the effect of enhancing immune system activity.

Based on the above findings, OX40 has become an important target for immunotherapy, and numerous preclinical and clinical studies suggest that OX40 may be an important target for tumor immunotherapy. Interestingly, recent studies have also found the role of OX40 signaling pathway in inhibiting hepatitis B virus infection [10], suggesting that OX40 agonists may be used as apotential means for antiviral infections, such as the treatment of hepatitis B patients.

Interferons is a class of multifunctional glycoproteins with a high activity. On the one hand, interferon can exert a potent anti-tumor effect by regulating tumor cell proliferation, inhibiting tumor metastasis and angiogenesis, and activating anti-tumor immune response; on the other hand, by regulating the human immune system, interferon has an important clinical application value in antiviral application, for example, interferon has become one of important means for clinical treatment of hepatitis B virus infection.

Both OX40 agonist and interferon have an important application value or potential in anti-tumor and anti-viral applications, but the existing evidence suggests that they have deficiencies in patient responsiveness and efficacy. Therefore, there is a demand for OX40 agonist and interferon showing better therapeutic effects.

### Contents of the Invention

It is an objective of the present invention to provide a fusion protein comprising an antibody or antigen-binding fragment thereof that specifically binds to human OX40, and a human interferon. The present invention also provides use of the fusion protein for treating tumors and/or viral infections.

To achieve the above objectives, the present invention adopts the following technical solutions.

In one aspect, the present invention provides a fusion protein comprising:
a) an antibody or antigen-binding fragment thereof that specifically binds to human OX40; and
b) a human interferon;
wherein the human interferon is linked to the carboxyl- or amino-terminus of the light or heavy chain of the antibody directly or via a peptide linker.

In the fusion protein according to the present invention, the antibody or antigen-binding fragment thereof that specifically binds to human OX40 comprises:
an antibody heavy chain variable region comprising VH CDR1 having the amino acid sequence of SEQ ID NO: 1, VH CDR2 having the amino acid sequence of SEQ ID NO: 2, and VH CDR3 having the amino acid sequence of SEQ ID NO: 3; and
an antibody light chain variable region comprising VL CDR1 having the amino acid sequence of SEQ ID NO: 4, VL CDR2 having the amino acid sequence of SEQ ID NO: 5, and VL CDR3 having the amino acid sequence of SEQ ID NO: 6.

In the fusion protein according to the present invention, the heavy chain variable region comprises the amino acid sequence as shown in SEQ ID NO: 7, and the light chain variable region comprises the amino acid sequence as shown in SEQ ID NO: 8.

In the fusion protein according to the present invention, the antibody or antigen-binding fragment thereof that specifically binds to human OX40 is a camelized single-domain antibody, scFv, a scFv dimer, BsFv, dsFv, dsFv2, dsFv-dsFv', a Fv fragment, Fab, Fab', F(ab')2, a ds diabody, a nanobody, a domain antibody, or a bivalent domain antibody.

In the fusion protein according to the present invention, the antibody further comprises a constant region of immunoglobulin, for example, a constant region of human IgG1, IgG2 or IgG4.

In the fusion protein according to the present invention, the human interferon is selected from the group consisting of human interferon type I, human interferon type II, and human interferon type III; preferably the human interferon is IFNα2a, IFNβ, IFNγ, IFNλ, or IFNα2b; more preferably, the human interferon is IFNα2b having the amino acid sequence as shown in SEQ ID NO: 9.

Further preferably, the human interferon is a IFNα2b mutant, which has one or more mutations selected from the group consisting of: T106A, R149A, A145G, A145D, R120A, and L117A, relative to the amino acid sequence as shown in SEQ ID NO: 9; more preferably, the IFNα2b mutant has one or more double mutations selected from the group consisting of: T106A/A145D, T106A/R149A, T106A/A145G, T106A/L117A, and T106A/R120A, relative to the amino acid sequence as shown in SEQ ID NO: 9.

In the fusion protein according to the present invention, the peptide linker is selected from the group consising of (G)ₙ, KESGSVSSEQLAQFRSLD, EGKSSGSGSESKST, GSAGSAAGSGEF, (GGGGS)ₙ, and (GGSGG)ₙ; preferably, the peptide linker is (GGGGS)ₙ; wherein n is an integer between 0 and 5; preferably, n is an integer between 1 and 3.

In the fusion protein according to the present invention, the fusion protein is selected from the group consisting of:
UMY02-L1, comprising the amino acid sequences as shown in SEQ ID NO: 10 and SEQ ID NO: 11, wherein the amino acid sequence as shown in SEQ ID NO: 10 represents the heavy chain of the antibody of OX40, the peptide linker and the human interferon, and the amino acid sequence as shown in SEQ ID NO: 11 represents the light chain of the antibody of OX40;
UMY02-L2, comprising the amino acid sequences as shown in SEQ ID NO: 12 and SEQ ID NO: 13, wherein the amino acid sequence as shown in SEQ ID NO: 12 represents the heavy chain of the antibody of OX40, and the amino acid sequence as shown in SEQ ID NO: 13 represents the light chain of the antibody of OX40, the peptide linker and the human interferon;
UMY02-L3, comprising the amino acid sequences as shown in SEQ ID NO: 14 and SEQ ID NO: 13, wherein the amino acid sequence as shown in SEQ ID NO: 14 represents the heavy chain of the antibody of OX40, and the amino acid sequence as shown in SEQ ID NO: 13 represents the light chain of the antibody of OX40, the peptide linker and the human interferon;
UMY02-L4, in which the heavy chain is as shown in SEQ ID NO: 14, and the light chain and the human interferon are as shown in SEQ ID NO: 13, without a peptide linker;
UMY02-L5, in which the heavy chain is as shown in SEQ ID NO: 14, and the light chain and the human interferon are as shown in SEQ ID NO: 13, with the peptide linker of GGGGS;
UMY02-L6, in which the heavy chain is as shown in SEQ ID NO: 14, and the light chain and the human interferon are as shown in SEQ ID NO: 13, with the peptide linker of (GGGGS)2;
UMY02-L7, in which the heavy chain is as shown in SEQ ID NO: 15, and the light chain and the human interferon are as shown in SEQ ID NO: 13, with the peptide linker of GGGGS;
UMY02-L8, in which the heavy chain is as shown in SEQ ID NO: 15, and the light chain and the human interferon are as shown in SEQ ID NO: 13, with the peptide linker of (GGGGS)2;
UMY02-L13, in which the heavy chain is as shown in SEQ ID NO: 15, and the light chain and the human interferon are as shown in SEQ ID NO: 13, with the peptide linker of (GGGGS)2, and the interferon has the mutation of T106A/A145D relative to SEQ ID NO: 9;
UMY02-L14, in which the heavy chain is as shown in SEQ ID NO: 15, and the light chain and the human interferon are as shown in SEQ ID NO: 13, with the peptide linker of (GGGGS)2, and the interferon has the mutation of T106A/R149A relative to SEQ ID NO: 9;
UMY02-L15, in which the heavy chain is as shown in SEQ ID NO: 15, and the light chain and the human interferon are as shown in SEQ ID NO: 13, with the peptide linker of (GGGGS)2, and the interferon has the mutation of T106A/R120A relative to SEQ ID NO: 9;
UMY02-L16, in which the heavy chain is as shown in SEQ ID NO: 15, and the light chain and the human interferon are as shown in SEQ ID NO: 13, with the peptide linker of (GGGGS)2, and the interferon has the mutation of T106A/A145G relative to SEQ ID NO: 9;
UMY02-L17, in which the heavy chain is as shown in SEQ ID NO: 15, and the light chain and the human interferon are as shown in SEQ ID NO: 13, with the peptide linker of (GGGGS)3, and the interferon has the mutation of T106A/R149A relative to SEQ ID NO: 9;
UMY02-L18, in which the heavy chain is as shown in SEQ ID NO: 15, and the light chain and human interferon are as shown in SEQ ID NO: 13, with the peptide linker of (GGGGS)2, and the interferon has the mutation of T106A/L117A relative to SEQ ID NO: 9; and
The fusion proteins OX40 IFN-α2a, OX40-IFNβ, OX40-IFNγ, and OPX40-IPNλ3 in which the heavy chains of OX40 IFN-α2a, OX40-IFNβ, OX40-IFNy, and OX40-IFNλ3 are as shown in SEQ ID NO: 15; the light chain, the peptide linker and the interferon of OX40 IFN-α2a are as shown in SEQ ID NO: 16; the light chain, the peptide linker and the interferon of OX40 IFN-β are as shown in SEQ ID NO: 17; the light chain, the peptide linker and the interferon of OX40 IFN-γ are as shown in SEQ ID NO: 18; and the light chain, the peptide linker and the interferon of OX40 IFN-λ3 are as shown in SEQ ID NO: 19.

In another aspect, the present invention provides an isolated polynucleotide encoding the fusion protein.

In yet another aspect, the present invention provides a vector comprising the isolated polynucleotide.

In yet another aspect, the present invention provides a host cell comprising the vector.

The present invention also provides a method of expressing the fusion protein comprising culturing the host cell under conditions capable of expressing the isolated polynucleotide.

The present invention also provides a kit containing the fusion protein.

The present invention also provides a pharmaceutical composition comprising the fusion protein and a pharmaceutically acceptable carrier.

The present invention also provides use of the fusion protein in preparing a medicament for treating a condition benefiting from enhancing an immune response and/or exposure to an interferon.

Preferably, the condition is cancer or viral infection, such as hepatitis B virus infection.

The present invention provides a method of treating a condition benefiting from enhancing an immune response and/or exposure to an interferon, comprising administering to a subject in need thereof a therapeutically effective amount of the fusion protein; preferably, the condition is cancer or viral infection, such as hepatitis B virus infection.

The present invention provides a fusion protein for treating a condition benefiting from enhancing an immune response and/or exposure to an interferon; preferably, the condition is cancer or viral infection, such as hepatitis B virus infection.

The present invention provides a fusion protein of an OX40 agonistic antibody and an interferon, which combines their different action mechanisms simultaneously and produces a synergistic enhancement effect: on the one hand, when the interferon moiety in the fusion protein binds to the surface of a cell that highly expresses interferon receptors (e.g., tumor or virus-infected cell), it is possible to enhance the activity of OX40 activating antibody in a receptor-mediated manner, thereby better enhancing the activity of immune system; on the other hand, compared with an interferon molecule, the half-life of the fusion protein of the present invention is greatly prolonged, so that the fusion protein can be administered clinically at a lower frequency, and has great advantages compared with the current interferons which need to be injected clinically every day.

In summary, the fusion protein of the present invention is promising to show unique efficacy and compliance advantages in antitumor and antiviral therapy.

### Brief Description of the Drawings

The embodiments of the present invention will be described below in conjunction with the accompanying drawings, wherein:
FIG. 1 shows the schematic diagram of structures of the fusion proteins according to the present invention;
FIG. 2 shows the results of ELISA binding assay of the fusion proteins according to the present invention to human OX40 protein;
FIG. 3 shows the FACS assay results showing that the MT01-L1 antibody binds to both human and Cynomolgus monkey OX40, but not to mouse OX40;
FIG. 4 shows the proliferation inhibitory effects of the fusion proteins according to the present invention on Daudi cells;
FIG. 5 shows that the fusion proteins according to the present invention activate the activity of NF-κB signaling pathway in Jurkat cells;
FIG. 6 shows that the fusion proteins according to the present invention can promote the activity of the MT01-C1 antibody for activating OX40 signaling pathway. A OX40 monoclonal antibody (mAb) (MT01-C1 or MT01-C1 (G2)) alone or the interferon molecule IFNα2b has no or only a weak activation effect on the OX40 signaling pathway in Jurkat cells in this experimental system (6a). However, the fusion proteins (UMY02-L1, UMY02-L2 and UMY02-L3) have a significant activation activity on the signaling pathway of OX40 under the same conditions (6b);
FIG. 7 shows the pharmacokinetic profiles of UMY02-L1 and UMY02-L3 on mice (7a). For comparison, FIG. 7b shows the pharmacokinetic profile of MT01-C1 on mice; and
FIG. 8 shows the killing effects of the fusion proteins according to the present invention on tumor cells.

### Best Modes for Practicing the Invention

The following description of the present application is merely illustrative of various embodiments of the present application. Therefore, the specific embodiments discussed herein should not be construed as limiting the scope of the present application. Numerous equivalents, changes and modifications will readily occur to those skilled in the art without departing from the scope of the present application, and it is to be understood that such equivalents are encompassed within the scope of the present invention. All documents, including publications, patents, and patent applications, cited in this application are hereby incorporated by reference in their entirety.

### Definitions

The term "antibody" in the present invention encompasses any immunoglobulin, monoclonal antibody, polyclonal antibody, multispecific antibody, or bispecific (bivalent) antibody that can bind to a specific antigen. A native intact antibody comprises two heavy chains and two light chains. Each heavy chain consists of a variable region, a first constant region, a second constant region and a third constant region, and each light chain consists of a variable region and a constant region. Mammalian heavy chains can be classified as α, δ, ε, γ, and µ, and mammalian light chains can be classified as λ or κ. An antibody is of "Y" type, and the neck of the Y-type structure consists of a second and a third constant regions of the two heavy chains, which are bound by disulfide bonds. Each arm of the "Y" structure comprises a variable region and a first constant region of one of the heavy chains, which bind to a variable region and a constant region of one of the light chains. The variable regions of the light and heavy chains determine antigen binding. The variable region of each chain comprises three hypervariable regions, termed complementarity determining regions (CDRs). The CDRs of the light chain (L) include LCDR1, LCDR2, and LCDR3, and the CDRs of the heavy chain (H) include HCDR1, HCDR2, and HCDR3. The CDR boundaries of the antibody and antigen-binding fragment disclosed in the present invention may be named or recognized by Kabat, Chothia or Al-Lazikani nomenclature (AI-Lazikani, B., Chothia, C., Lesk, A.M., J. Mol. Biol., 273(4): 927(1997); Chothia, C. et. al, J. Mol. Biol., 186(3): 651-63(1985); Chothia, C. and Lesk, A.M., J. Mol. Biol., 196: 901 (1987); Chothia, C. et. al, Nature, 342(6252): 877-83 (1989); Kabat, E.A. et. al, National Institutes of Health, Bethesda, Md. (1991)). The three CDRs are separated by contiguous flanking regions called framework regions (FR), which are more highly conserved than the CDRs and form a scaffold to support the hypervariable loop. The constant regions of the heavy and light chains are independent of antigen binding, but have multiple effector functions. Antibodies can be divided into several classes depending on the amino acid sequence of the constant region of heavy chain. Antibodies can be divided into five major classes or isomers depending on whether they comprise α, δ, ε, γ, and µ heavy chains, respectively: IgA, IgD, IgE, IgG and IgM. Several major antibody classes can further be divided into subclasses, such as IgG1 (γ1 heavy chain), IgG2 (y2 heavy chain), IgG3 (γ3 heavy chain), IgG4 (γ4 heavy chain), IgA1 (α1 heavy chain), IgA2 (α2 heavy chain), or etc.

The term "antigen-binding fragment" as used herein refers to an antibody fragment formed from an antibody portion comprising one or more CDRs, or any other antibody fragment that binds to an antigen but does not have an intact antibody structure. Examples of antigen-binding fragments include, but are not limited to, a diabody, Fab, Fab', F(ab')2, a Fv fragment, a disulfide-stabilized Fv fragment (dsFv), (dsFv)2, bispecific dsFv (dsFv-dsFv'), a disulfide-stabilized diabody (ds diabody), a single chain antibody molecule (scFv), a scFv dimer (a bivalent diabody), a bivalent single-chain antibody (BsFv), a multispecific antibody, a camelized single domain antibody, a nanobody, a domain antibody, and a bivalent domain antibody. The antigen-binding fragment can bind to the same antigen as the parent antibody. In some embodiments, the antigen-binding fragment may comprise one or more CDRs from a particular human antibody grafted to a framework region from one or more different human antibodies.

An "Fab" fragment of an antibody refers to a portion of antibody molecule consisting of one light chain (comprising the variable and constant regions), and one heavy chain variable and partial constant regions, which are bound together by disulfide bonds.

An "Fab'" fragment refers to a Fab fragment comprising a portion of hinge region.

"F(ab')2" refers to a dimer of Fab.

An Fc fragment of antibody is responsible for a variety of different effector functions, such as ADCC and CDC, but is not involved in antigen binding.

An "Fv" fragment of antibody refers to a minimum antibody fragment that comprises the entire antigen binding site. An Fv fragment consists of a light chain variable region and a heavy chain variable region.

"Fusion protein" refers to a recombinant protein that genetically links a cDNA encoding a protein of interest to a cDNA encoding an antibody or antibody fragment, and is expressed in a eukaryotic or prokaryotic expression system.

"Linker" refers to a peptide chain consisting of 1-50 amino acids forming a peptide bond, or a derivative thereof, the N- and C-termini of which form a covalent bond with either the anti-OX40 antibody or the interferon, respectively, thereby binding the anti-OX40 antibody to the interferon. The anti-OX40 antibody and the interferon may be integrated by binding to the N-terminus or C-terminus of the interferon, respectively, at the C-terminus or N-terminus side of the heavy or light chain of the anti-OX40 antibody via a linker sequence or directly using a peptide bond. As a preferred embodiment of the fusion protein of interferon with anti-OX40 antibody, the following may be listed: a fusion protein obtained by binding the C-terminus of the heavy chain or light chain of the anti-OX40 antibody to the N-terminus of the interferon via a linker sequence; alternatively, a fusion protein obtained by binding the N-terminus of the heavy or light chain of the anti-OX40 antibody to the C-terminus of the interferon via a linker sequence.

"Single chain Fv antibody" or "scFv" refers to an engineered antibody formed by binding a light chain variable region to a heavy chain variable region directly or via a peptide chain (Huston JS et al., Proc Natl Acad Sci USA, 85: 5879 (1988)).

"Single chain antibody Fv-Fc" or "scFv-Fc" refers to an engineered antibody consisting of scFv bound to an Fc fragment of antibody.

"camelized single domain antibody", "heavy chain antibody" or "HCAb (Heavy-chain-only antibody)" all refers to an antibody comprising two VH domains and no light chain (Riechmann L. and Muyldermans S., J Immunol Methods, 231(1-2): 25-38 (1999); Muyldermans S., J Biotechnol, 74(4): 277-302 (2001); WO94/04678; WO94/25591; U.S. Patent No. 6,005,079). Heavy chain antibodies were originally derived from the family Camelidae (camels, dromedaries and llamas). Despite the absence of light chain, camelized antibodies have a confirmed antigen binding repertoire (Hamers Casterman C. et. al, Nature 363(6428): 446-8(1993); Nguyen VK. et. al, "Heavy-chain antibodies in Camelidae: a case of evolutionary innovation, Immunogenetics. 54 (1): 39-47 (2002); Nguyen VK. et. al, Immunology. 109(1): 93-101 (2003)). The variable region (VH domain) of heavy chain antibody is the known minimum antigen binding unit produced by acquired immune (Koch-Nolte F. et al., FASEB J. 21(13): 3490-8. Epub (2007)).

"Nanobody" refers to an antibody fragment consisting of a VH domain and two constant regions CH2 and CH3 from an heavy chain antibody.

A "diabody" comprises a small antibody fragment with two antigen binding sites, wherein the fragment comprises a VH domain linked to a VL domain on the same polypeptide chain (see Holliger P. et al., Proc Natl Acad Sci USA. 90(14): 6444-8 (1993); EP404097; WO93/11161). By using a linker that is too short to allow pairing between the two domains on the same chain, the two domains are forced to pair with the complementary domains of another chain and create two antigen-binding sites. The two antibody binding sites may targetedly bind to the same or different antigens (or antigenic epitopes).

"Domain antibody" refers to an antibody fragment that comprisres only one heavy chain variable region or one light chain variable region. In some cases, two or more VH domains are covalently bound by a polypeptide linker to form a bivalent domain antibody. The two VH domains of bivalent domain antibody may target the same or different antigens.

In some embodiments, "(dsFv)2" comprises three peptide chains: two VH groups linked by a polypeptide linker and linked to two VL groups by disulfide bonds.

In some embodiments, a "bispecific ds diabody" comprises VL1-VH2 (linked by two polypeptide linkers) and VH1-VL2 (also linked by two polypeptide linkers), both of which are linked by disulfide bonds between VH1 and VL1.

A "bispecific dsFv" or "dsFv-dsFv" comprises three polypeptide chains: VH1-VH2 groups, the heavy chains of which are linked via a polypeptide linker (e. g., a long elastic linker) and are linked to the VL1 and VL2 groups via disulfide bonds, respectively; and each pair of heavy and light chains paired by disulfide bonds has a different antigen specificity.

In some embodiments, a "scFv dimer" is a bivalent diabody or a bivalent single-chain antibody (BsFv) comprising two dimerized VH-VL (linked by a polypeptide linker) groups, wherein the VH of the two groups cooperate with the VL of the other group to form two binding sites that can targetedly bind to the same antigen (or antigenic epitope) or to different antigens (or antigenic epitopes). In other embodiments, a "scFv dimer" is a bispecific diabody comprising interconnected V_{L1}-V_{H2} (linked by a polypeptide linker) and V_{H1}-V_{L2} (linked by a polypeptide linker), wherein V_{H1} cooperates with V_{L1}, V_{H2} cooperates with V_{L2}, and each cooperating pair has a different antigen specificity.

The term "fully human" as used herein, when used in reference to an antibody or antigen-binding fragment, refers to the antibody or antigen-binding fragment having or consisting of an amino acid sequence corresponding to the amino acid sequence of an antibody produced by a human or human immune cell, or derived from a non-human source such as a transgenic non-human animal utilizing a human antibody repertoire, or other sequences encoding human antibodies. In some embodiments, a fully human antibody does not comprise amino acid residues (particularly antigen-binding residues) derived from a non-human antibody.

The term "humanized" as used herein, when used in reference to an antibody or antigen-binding fragment, refers to an antibody or antigen-binding fragment that comprises CDRs derived from a non-human animal, FR regions derived from a human, and constant regions derived from a human (where applicable). Since a humanized antibody or antigen-binding fragment has a reduced immunogenicity, it can be used as a therapeutic agent for human in some embodiments. In some embodiments, the non-human animal is a mammal, such as mouse, rat, rabbit, goat, sheep, guinea pig, or hamster. In some embodiments, the humanized antibody or antigen-binding fragment consists essentially of human sequences, except that the CDR sequences are of non-human origin. In some embodiments, the human-derived FR regions may comprise the same amino acid sequence as the human antibody from which they are derived, or they may comprise some amino acid alterations, for example, no more than 10, 9, 8, 7, 6, 5, 4, 3, 2, or 1 amino acid alteration. In some embodiments, the amino acid alteration may be present only in the heavy chain FR region, only in the light chain FR region, or in both chains. In some preferred embodiments, the humanized antibody comprises human FR1-3 and human JH and JK.

As used herein, the term "chimeric" refers to an antibody or antigen-binding fragment having a portion of heavy and/or light chain derived from one species and the remainder of heavy and/or light chain derived from different species. In one illustrative example, the chimeric antibody may comprise a constant region derived from a human and a variable region derived from a non-human animal, such as mouse.

The term "OX40" refers to the receptor which binds to OX40L. It is a type I membrane protein belonging to the TNF receptor family, also named ACT-4, OX40L receptor, CD134 antigen, ACT35 antigen, or TNFRSF4. It has a molecular weight of 50 kDa and is registered in SwissProt with an accession number P43489.

As used herein, "human interferon" refers to a class of highly active, multifunctional secretory glycoproteins with antiviral, immunomodulatory, antitumor effects, and etc. It can be divided into type I, type II and type III IFNs according to the gene sequence, receptor specificity, and etc. The human type I IFN consists of 13 subtypes of IFNα, and IFNβ, IFNε, IFNκ and IFNω. Type I IFN has common cell surface receptor IFNAR composed of two subunits, namely IFNAR1 and IFNAR2. There is only one type II interferon, namely IFNγ. IFNγ has cell surface receptor IFNGR composed of two subunits, namely 1FNGR1 and IFNGR2. Type III IFN consists of IFNλ1, IFNλ2, IFNλ3 and IFNλ4. The interferon in the fusion protein of the present application also includes interferon variants known in the art.

As used herein, "specific binding" refers to a non-random binding reaction between two molecules, such as the reaction between an antibody and an antigen. In some embodiments, the antibody or antigen-binding fragment thereof of the present application specifically binds to human and/or monkey OX40 with a binding affinity (K_{D}) ≤10⁻⁶ M. K_{D} in this application refers to a ratio of the dissociation rate to the binding rate (k_{off}/kₒₙ), which can be determined by surface plasmon resonance, for example using an instrument such as Biacore.

As used herein, "UMY02-L1" refers to a fusion protein having the heavy chain, the peptide linker and the human interferon as shown in SEQ ID NO: 10, and the light chain as shown in SEQ ID NO. 11, wherein the human interferon IFNα2b as shown in SEQ ID NO: 9 is linked to the carboxyl-terminus of the heavy chain via the peptide linker.

As used herein, "UMY02-L2" refers to a fusion protein having the heavy chain as shown in SEQ ID NO: 12, and the light chain, the peptide linker and the human interferon as shown in SEQ ID NO: 13, wherein the human interferon IFNα2b as shown in SEQ ID NO: 9 is linked to the carboxyl-terminus of the light chain via the peptide linker.

As used herein, "UMY02-L3" refers to a fusion protein having the heavy chain as shown in SEQ ID NO: 14, and the light chain, the peptide linker and the human interferon as shown in SEQ ID NO: 13, wherein the human interferon IFNα2b as shown in SEQ ID NO. 9 is linked to the carboxyl-terminus of the light chain via the peptide linker.

As used herein, "MT01-C1" refers to a monoclonal antibody, which has the same VH (SEQ ID NO: 7) and VL (SEQ ID NO: 8) sequences as UMY02-L1, UMY02-L2 and UMY02-L3, and the heavy and light chain constant regions being human IgG1 and κ chain, respectively.

As used herein, "MT01-C1(G2)" refers to a monoclonal antibody, which has the same VH (SEQ ID NO: 7) and VL (SEQ ID NO: 8) sequences as UMY02-L1, UMY02-L2 and UMY02-L3, and the heavy and light chain constant regions being human IgG2 and κ chain, respectively.

As used herein, "MT01-L1" refers to a human-mouse chimeric antibody, which has the same heavy and light chain CDR sequences (SEQ ID NO: 1-6) as UMY02-L1, UMY02-L2 and UMY02-L3.

"MT01-C1", "MT01-C1 (G2)", and "MT01-L1" are from the Chinese Patent 201711476160.3, and their specific sequences are as shown in Table 1 and the sequence listing, all of which are incorporated herein by reference in their entirety.

As used herein, when applied to an amino acid sequence, "conservative substitution" refers to the substitution of one amino acid residue with another amino acid residue having a side chain with similar physical and chemical properties. For example, conservative substitution may be conducted among amino acid residues having a hydrophobic side chain (e.g., Met, Ala, VaL, Leu, and Ile), amino acid residues having a neutral hydrophilic side chain (e.g., Cys, Ser, Thr, Asn, and Gln), amino acid residues having an acidic side chain (e.g., Asp and Glu), amino acid residues having a basic side chain (e.g., His, Lys, and Arg), or amino acid residues having an aromatic side chain (e.g., Trp, Tyr and Phe). It is known in the art that a conservative substitution generally does not cause a significant change in the conformational structure of a protein, and thus is capable of retaining the biological activity of the protein.

When applied to an amino acid sequence (or a nucleic acid sequence), "percent sequence identity" refers to a percentage of amino acid (or nucleic acid) residues in a candidate sequence that are identical to those of a reference sequence, relative to the amino acid (or nucleic acid) residues in the candidate sequence during sequence alignment, and if necessary, after introducing gaps to maximize the number of identical amino acids (or nucleic acids). A conservative substitution of amino acid residue may or may not be considered as an identical residue. Percent sequence identity of amino acid (or nucleic acid) sequences can be determined by aligning sequences through tools disclosed in the art. A person skilled in the art may use the default parameters of the tools or adjust the parameters appropriately according to the needs of the alignment, for example by choosing an appropriate algorithm.

As used herein, "T cells" include CD4+ T cells, CD8+ T cells, T helper 1 cells, T helper 2 cells, T helper type 17 T cells, and suppressor T cells.

As used herein, "effector function" refers to a biological activity of Fc region of an antibody in binding to its effectors, e.g., C1 complex and Fc receptors. Exemplary effector functions include complement-dependent cytotoxicity (CDC) induced by the interaction between an antibody and Clq on a C1 complex, antibody-dependent cell-mediated cytotoxicity (ADCC) induced by the binding of Fc region of an antibody to the Fc receptors on effector cells, and phagocytosis.

As used herein, "cancer" or "cancerous condition" refers to any medical condition mediated by the growth, proliferation or metastasis of tumors or malignant cells and eliciting solid and non-solid tumors such as leukemia. A "tumor" in the present invention refers to a solid substance of tumor and/or malignant cells.

As used herein, "viral infection" refers to a pathogenic process in which viruses invade a human body through various pathways and proliferate in human cells, resulting in damage to the body, including chronic viral infections, such as viral infection of hepatitis B virus, hepatitis C virus, herpes virus, Epstein-Barr virus, HIV, cytomegalovirus, herpes simplex virus type I, herpes simplex virus type 2, human papilloma virus, or adenovirus, Kaposi's sarcoma-associated herpesvirus epidemics, and infection of Torquetenovirus, JC virus, or BK virus.

"Treatment" or "therapy" of a condition includes preventing or reducing the condition, reducing the rate of rise or development of the condition, reducing the risk of developing the condition, preventing or delaying the development of symptoms associated with the condition, reducing or terminating symptoms associated with the condition, causing a complete or partial reversal of the condition, curing the condition, or a combination thereof. For cancers, "treatment" or "therapy" may refer to inhibiting or slowing growth, proliferation or metastasis of tumors or malignant cells, or some combination thereof. For tumors, "treatment" or "therapy" includes clearing all or a portion of tumors, inhibiting or slowing growth and metastasis of tumors, preventing or slowing the development of tumors, or some combination thereof.

An "isolated" material has been artificially altered from its natural state. If an "isolated" substance or component occurs in nature, it has been altered or removed from its original state, or both. For example, a polynucleotide or polypeptide naturally occurring in a living animal is not isolated, but may be considered "isolated" if the polynucleotide or polypeptide is sufficiently isolated from the materials with which it coexists in its native state and exists in a sufficiently pure state. In some embodiments, the antibody and antigen-binding fragment are at least 90%, 93%, 95%, 96%, 97%, 98%, 99% pure as determined by electrophoresis (e.g., SDS-PAGE, isoelectric focusing, capillary electrophoresis), or chromatography (e.g., ion exchange chromatography or reverse phase HPLC).

A "vector" in the present invention refers to a vehicle into which a polynucleotide encoding a protein can be operably inserted for enabling the protein to be expressed. The vector can be used to transform, transduce or transfect a host cell, such that the genetic elements carried by the vector are expressed in the host cell. For example, the vectors include: plasmids, phagemids, cosmids, artificial chromosomes such as yeast artificial chromosome (YAC), bacterial artificial chromosome (BAC) or PI-derived artificial chromosome (PAC), bacteriophages such as λ bacteriophage or M13 bacteriophage, animal viruses, and etc. Animal viral species used as vectors include retroviruses (including lentiviruses, adenoviruses, adeno-associated viruses, herpes viruses (e.g., herpes simplex viruses), poxviruses, baculoviruses, papillomaviruses, papovaviruses (e.g., SV40)). The vector may comprise a variety of elements that control expression, including a promoter sequence, a transcription initiation sequence, an enhancer sequence, a selection element, and a reporter gene. Further, the vector may also comprise an origin of replication. The vector may also comprise a component that facilitates the vetor to enter into cells, including, but not limited to, viral particle, liposome, or protein shell.

A "host cell" in the present invention refers to a cell into which an exogenous polynucleotide and/or a vector are introduced.

A "therapeutically effective amount" or an "effective dose" in the present invention refers to a dose or a concentration of a drug effective to treat a disease. For example, for the use of the antibody or antigen-binding fragment thereof disclosed herein, a therapeutically effective amount means that at that dose or concentration, the antibody or antigen-binding fragment can clear all or a portion of tumors, inhibit or slow the growth of tumors, inhibit metastasis of tumor cells, alleviate any symptoms or markers associated with tumors or cancerous conditions, prevent or delay the development of tumors or cancerous conditions, inhibit or eliminate viruses or virus infected cells, or some combination thereof.

"Pharmaceutically acceptable" means that the carrier, solvent, diluent, adjuvant, and/or salt referred to is generally chemically and/or physically compatible with the other ingredients in the formulation and physiologically compatible with the recipient.

### Regarding the fusion protein of the present invention

In some embodiments, the exemplary fusion proteins UMY02-L1, UMY02-L2, and UMY02-L3 are provided herein.

It will be appreciated by those skilled in the art that the foregoing CDR sequences may be modified to comprise one or more amino acid substitutions, thereby resulting in increased biological activities, e.g., an increased binding affinity to human OX40. For example, a library of antibody variants (e.g., Fab or FcFv variants) can be produced and expressed using phage display technique, followed by screening for the antibodies having affinity to human OX40. In another example, computer software can be used to simulate the binding of the antibody to human OX40 and identify the amino acid residues on the antibody that form a binding interface. Substitutions of these residues may be avoided to prevent a decrease in binding affinity, or these residues may be targeted for substitution to form a stronger binding. In some embodiments, at least one (or all) substitution in a CDR sequence is a conservative substitution.

In some embodiments, the fusion protein and antigen-binding fragment comprise one or more CDR sequences having at least 80% (e.g., at least 85%, 88%, 90%, 91%, 92%, 93%, 94%, 95%, 96%, 97%, 98%, or 99%) sequence identity with the sequences set forth for the exemplary fusion proteins UMY02-L1, UMY02-L2, and/or UMY02-L3 provided herein, while retaining the binding affinity to human OX40 similar to or even higher than that of its parent antibody. The parent antibody has substantially the same sequence, but its corresponding CDR sequences have 100% sequence identity to the sequences listed.

In some embodiments, the fusion protein described herein is capable of specifically binding to human OX40 with a binding affinity (K_{D}) of ≤10⁻⁷ M, as measured by surface plasmon resonance. The binding affinity value can be expressed as a K_{D} value, which is calculated as the ratio of the dissociation rate to the association rate (k_{off}/kₒₙ) when the binding of an antigen to an antigen-binding molecule reaches an equilibrium. The antigen binding affinity (e.g., K_{D}) may be appropriately determined by any suitable methods known in the art, including, for example, a plasma resonance binding method using an instrument such as Biacore.

In some embodiments, the fusion protein described herein binds to human OX40 with an EC50 (i.e., half binding concentration) of 10 ng/mL to 10 (µg/mL. The binding of the antibody or the fusion protein to human OX40 can be determined by the methods known in the art, for example, sandwich methods such as ELISA, Western blot, FACS or other binding assays. In an illustrative example, an antibody to be tested (i.e., a primary antibody) is bound to immobilized human OX40 or cells expressing human OX40, followed by washing away unbound antibody and introducing a labeled secondary antibody that is capable of binding to the primary antibody, thus capable of detecting the bound secondary antibody. The detection can be performed on a microplate reader when immobilized OX40 is used, or can be performed using FACS assay when the cells expressing human OX40 are used.

In some embodiments, the fusion protein described herein binds to human OX40 with an EC50 (i.e., 50% effective concentration) of 0.1 (µg/mL to 10 (µg/mL (determined using FACS assay).

In some embodiments, the fusion protein described herein may activate human OX40 signaling pathway by either FcR-mediated or interferon receptor-mediated mode, thus providing the biological activities, including, for example, inducing cytokine production by activated T cells (e.g., CD4+ T cells and CD8+ T cells), inducing proliferation of activated T cells (e.g., CD4+ T cells and CD8+ T cells), and reversing the inhibitory function of regulatory Treg.

The fusion protein is specific to human OX40. In some embodiments, the fusion protein does not bind to murine OX40, but binds to monkey OX40 with a binding affinity similar to that of human OX40. For example, the binding of the monoclonal antibody MT01-L1 with the same CDR sequences as the fusion protein of the present invention to mouse OX40 cannot be detected by conventional binding assay methods, such as FACS assay, while FACS detects that MT01-L1 binds to both monkey OX40 and human OX40.

In some embodiments, the fusion protein has a constant region of IgG2 isotype with reduced or eliminated effector functions. Effector functions such as ADCC and CDC can cause cytotoxicity to cells expressing OX40. Some normal cells are capable of expressing OX40. To avoid potentially undesirable toxicity to these normal cells, some embodiments of the antibody of the present invention have reduced or even eliminated effector functions. A number of assays are known for assessing ADCC or CDC activity, such as Fc receptor binding assay, complement Clq binding assay, and cell lysis assay, which can be readily selected by those skilled in the art. Without wishing to be bound by theory, it is believed that the antibody having reduced or eliminated effector functions such as ADCC and CDC does not cause or minimize cytotoxicity to cells expressing OX40 (e.g., those normal cells), thus avoiding undesirable side effects.

In some embodiments, the fusion protein described herein has an extended duration of action in an organism than an interferon molecule. This is because the fusion protein has a longer half-life and drug retention time in animals. This property is beneficial to reducing the number of medications by patients and improving the efficacy of drugs.

In some embodiments, the fusion protein described herein has reduced side effects. For example, the anti-OX40 antibody and antigen-binding fragment thereof may have a fully human IgG sequence and therefore be less immunogenic than a humanized antibody. As another example, the fusion protein and antigen-binding fragment thereof may have the form of IgG2 or IgG4 to eliminate ADCC and CDC.

In some embodiments, the fusion protein described herein is advantageous in that it can be used in combination with immunogenic substances, such as tumor cells, purified tumor antigens, and the cells transfected with the encoded immunostimulatory factors, and tumor vaccines. In addition, the anti-OX40 antibody and antigen-binding fragment thereof may be included in combination therapies, including standard chemotherapy and radiation therapy, target-based small molecule therapy, and other emerging immune checkpoint modulator therapy. In some embodiments, the antibody and antigen-binding fragment thereof can be used as a basic molecule for an antibody-drug conjugate, and a bispecific or multivalent antibody.

In some embodiments, the fusion protein or antigen-binding fragment thereof described herein is a camelized single chain domain antibody, a diabody, scFv, a scFv dimer, BsFv, dsFv, (dsFv)2, dsFv-dsFv', a Fv fragment, Fab, Fab', F(ab')2, a ds diabody, a nanobody, a domain antibody, or a bivalent domain antibody.

In some embodiments, the fusion protein described herein comprises an immunoglobulin constant region. In some embodiments, the immunoglobulin constant region comprises a heavy chain and/or a light chain constant region. The heavy chain constant region comprises a CHI, CH1-CH2 or CH1-CH3 region. In some embodiments, the immunoglobulin constant region may further comprise one or more modifications to achieve the desired properties. For example, the constant region may be modified to reduce or eliminate one or more effector functions to enhance FcRn receptor binding or to introduce one or more cysteine residues.

In some embodiments, the antibody and antigen-binding fragment thereof further include conjugates. It is contemplated that the antibody or antigen-binding fragment thereof of the present invention may be linked to a variety of conjugates (see, e.g., "Conjugate Vaccines", Contributions to Microbiology and Immunology, J. M. Cruse and R. E. Lewis. Jr. (eds.), Carger Press, New York (1989)). These conjugates may be linked to the antibody or antigen conjugate by covalent binding, affinity binding, intercalation, coordinate binding, complexation, binding, mixing, addition, or other means. In some embodiments, the antibody and antigen-binding fragment disclosed herein can be engineered to contain specific sites other than epitope-binding moieties that can be used to bind one or more conjugates. For example, such sites may comprise one or more reactive amino acid residues, such as cysteine residues and histidine residues, to facilitate covalent attachment to the conjugate. In some embodiments, the antibody may be linked to the conjugate indirectly, or via another conjugate. For example, the antibody or antigen-binding fragment thereof can bind to biotin and then indirectly bind to a second conjugate, which is linked to avidin. The conjugate may be a detectable label, a pharmacokinetically modified moiety, a purified moiety, or a cytotoxic moiety. Examples of detectable labels may include fluorescent labels (e.g., fluorescein, rhodamine, dansyl, phycoerythrin or Texas red), enzyme substrate labels (e.g., horseradish peroxidase, alkaline phosphatase, luciferase, glucoamylase, lysozyme, glucose oxidase or β-D galactosidase), stable or radioactive isotopes, chromophore moieties, digoxin, biotin/avidin, DNA molecules or gold for detection. In some embodiments, the conjugate may be a pharmacokinetically modified moiety, such as PEG, which helps to extend the half-life of the antibody. Other suitable polymers include, for example, carboxymethylcellulose, dextran, polyvinyl alcohol, polyvinylpyrrolidone, and ethylene glycol/propylene glycol copolymers, etc. In some embodiments, the conjugate may be a purified moiety, such as a magnetic bead. A "cytotoxic moiety" can be any agent that is harmful to a cell or that may damage or kill a cell. Examples of cytotoxic moieties include, but are not limited to, paclitaxel, cytochalasin B, gramicidin D, ethidium bromide, emetine, mitomycin, etoposide, teniposide, vincristine, vinblastine, colchicine, doxorubicin, daunorubicin, dihydroxyanthraquinone, mitoxantrone, mithramycin, actinomycin D, 1-dehydrotestosterone, glucocorticoids, procaine, tetracaine, lidocaine, propranolol, puromycin and analogue thereof, antimetabolites (e.g., methotrexate, 6-mercaptopurine, 6-thioguanine, cytarabine, 5-fluorouracil, dacarbazine), alkylating agents (e.g., nitrogen mustard, thiotepa, chlorambucil, melphalan, carmustine (BSNU) and lomustine (CCNU), cyclophosphamide, busulfan, dibromomannitol, streptozotocin, mitomycin C and cis-dichlorodiamine platinum (DDP), cisplatin, anthracycline antibiotics (e.g., daunorubicin (formerly daunomycin) and doxorubicin), antibiotics (e.g., dactinomycin (formerly known as actinomycin), bleomycin, mithramycin, and ampicillin (AMC)), and antimitotic agents (e.g., vincristine and vinblastine).

### Polynucleotide and recombinant method

Amino acid sequences of the fusion protein of the present application may be converted to corresponding DNA coding sequences using genetic engineering techniques well known in the art. Due to the degeneracy of genetic code, the transformed DNA sequences may not be completely identical, while the encoded protein sequences remain unchanged.

A vector comprising a polynucleotide encoding the fusion protein may be introduced into a host cell for cloning (amplification of DNA) or gene expression using recombinant techniques well known in the art. In another embodiment, the fusion protein can be prepared by homologous recombination methods well known in the art. A variety of vectors are available. The vector components typically include, but is not limited to, two or more selected from the group consisting of: a signal sequence, an origin of replication, one or more marker genes, an enhancer sequence, a promoter (for example: SV40, CMV, EF-1a), and a transcription termination sequence.

In some embodiments, the vector systems include mammalian, bacterial, and yeast systems, and will include plasmids such as, but not limited to, pALTER, pBAD, pcDNA, pCal, pL, pELpGEMEX, pGEX, pCLpCMV, pEGFP, pEGFT, pSV2, pFUSE, pVITRO, pVIVO, pMAL, pMONO, pSELECT, pUNO, pDUO, Psg5L, pBABE, pWPXL, pBI, p15TV-L, pPPro18, pTD, pRS420, pLexA, pACT2, and other vectors available from the laboratory or commercially available vectors. Suitable vectors may include plasmid or viral vectors (e.g., replication-defective retroviruses, adenoviruses, and adeno-associated viruses).

A vector comprising a polynucleotide encoding the fusion protein may be introduced into a host cell for cloning or gene expression. Host cells suitable for cloning or expressing the DNA in the vectors of the present invention are prokaryotic, yeast or the above-mentioned advanced eukaryotic cells. Prokaryotic cells suitable for use in the present invention include eubacteria, such as gram-negative or gram-positive bacteria, for example, Enterobacteriaceae such as E. coli, Enterobacter, Erwinia, Klebsiella, Proteus, Salmonella such as Salmonella typhimurium, Serratia such as Serratia marcescens, Shigella, and Bacillus such as Bacillus subtilis and Bacillus licheniformis, Pseudomonas such as Pseudomonas aeruginosa, and Streptomycete.

In addition to prokaryotic cells, eukaryotic microorganisms such as filamentous fungi or yeasts can also be used as host cells for cloning or expressing vectors encoding the fusion protein. Saccharomyces cerevisiae or baker's yeast is the most commonly used lower eukaryotic host microorganism. However, many other genera, species and strains are common and suitable for use in the present invention, such as Schizosaccharomyces pombe; Kluyveromyces hosts, such as Kluyveromyces lactis, Kluyveromyces fragilis (ATCC 12, 424), Kluyveromyces bulgaricus (ATCC 16, 045), Kluyveromyces wickerhamii (ATCC 24, 178), Kluyveromyces waltii (ATCC 56, 500), Kluyveromyces drosophila (ATCC 36, 906), Kluyveromyces thermotolerant and Kluyveromyces marxianus; Yarrowia lipolytica (EP 402, 226); Pichia pastoris (EP 183, 070); Candida; Trichoderma reesei (EP 244, 234); Neurospora; Schwanniomyces occidentalis, such as Schwanniomyces occidentalis; and filamentous fungi, such as Neurospora, Penicillium, Curvularia and Aspergillus, such as Aspergillus nidulans and Aspergillus niger.

The host cells suitable for expressing the glycosylated antibody or antigen-binding fragment thereof provided herein are derived from multicellular organisms. Examples of invertebrate cells include plant and insect cells. A variety of baculoviral strains and variants thereof and corresponding permissive insect host cells have been found, which are derived from hosts, such as Spodoptera frugiperda (caterpillar), Aedes aegypti (mosquito), Aedes albopictus (mosquito), Drosophila melanogaster (drosophila) and Bombyx mori. A variety of viral strains for transfection are publicly available, such as Autographa californica nuclear polyhedrosis virus and Bm-5 variants of Bombyx mori nuclear polyhedrosis virus, all of which can be used in the present invention, particularly for transfecting Spodoptera frugiperda cells. The plant cell cultures of cotton, corn, potato, soybean, petunia, tomato and tobacco can also be used as the hosts.

However, the most interesting host cells are vertebrate cells, and the culture of vertebrate cells (tissue culture) has become a routine practice. Examples of available mammalian host cells include monkey kidney CV1 line transformed by SV40 (COS-7, ATCC CRL 1651); human embryonic kidney cell line (293 or 293 cell subclone in suspension culture, Graham et al., Gen Virol. 36: 59 (1977)); baby hamster kidney cells (B blood, ATCC CCL 10); Chinese hamster ovary cells/-DHFR (CHO, Urlaub et al., Proc. Natl. Acad. Sci. USA 77: 4216 (1980)); mouse sertoli cells (TM4, Mather, Biol. Reprod. 23: 243-251 (1980)); monkey kidney cells (CV1, ATCC CCL 70); African green monkey kidney cells (VERO-76, ATCC CRL-1587); human cervical carcinoma cells (HELA, ATCC CCL 2); canine kidney cells (MDCK, ATCC CCL 34); Buffalo rat liver cells (BRL 3A, ATCC CRL 1442); human lung cells (W138, ATCC CCL75); human liver cells (Hep G2, HB 8065); mouse mammary tumor (MMT 060562, ATCC CCL51); TRI cells (Mather et al., Annals N. Y. Acad. Sci. 383: 44-68 (1982)); MRC5 cells; FS4 cells; and human hepatoma cell line (HepG2). In some preferred embodiments, the host cell is 293F cell.

The host cell is transformed with the above-mentioned expression or cloning vector that can produce the fusion protein, and then cultured in a conventional nutrient medium, which is suitable for inducing promoters, selecting transformed cells, or amplifying genes encoding target sequences after being modified.

The host cells used to produce the fusion protein in the present invention can be cultured in a variety of media known in the art. The media may also comprise any other necessary additives known in the art in a suitable concentration. The conditions of the media, such as temperature, pH and the like are those selected previously for expression of host cell, which are well known to those of ordinary skill.

When recombinant techniques are used, the antibody may be produced in the intracellular and periplasmic space, or directly secreted into the culture medium. If the antibody is produced intracellularly, the particulate debris of host cells or lysed fragments is first removed, for example, by centrifugation or sonication. Carter et al., Bio/Technology 10: 163-167(1992) describes a method for isolating an antibody secreted into the E. coli periplasmic space. Briefly, cell paste was thawed in the presence of uranyl acetate (pH 3.5), EDTA and phenylmethylsulfonyl fluoride (PMSF) for more than about 30 minutes. The cell debris was removed by centrifugation. If the antibody is secreted into the culture medium, the supernatant of expression system is typically first concentrated using a commercially available protein concentration filter, such as lAmicon or Millipore Pellicon ultrafiltration unit. In any of the foregoing steps, protease inhibitors such as PMSF may be added to inhibit protein degradation, and antibiotics may be added to prevent the growth of incidental contaminants.

Antibodies produced from the cells may be purified using purification methods such as hydroxyapatite chromatography, gel electrophoresis, dialysis, DEAE-cellulose ion exchange chromatography columns, ammonium sulfate precipitation, salting out, and affinity chromatography, wherein affinity chromatography is a preferred purification technique. The types of the antibody and the presence of any immunoglobulin Fc domain in the antibody determine whether protein A is suitable as an affinity ligand. Protein A can be used to purify antibodies based on human γ1, γ2 or γ4 heavy chain (Lindmark et al., J. Immunol. Meth. 62: 1-13 (1983)). Protein G is suitable for all murine isomers and human γ3 (Guss et al., EMBO J. 5: 1567-1575 (1986)). Agarose is the most commonly used matrix to which affinity ligands are attached, but other matrix may also be used. Mechanically stable matrix, such as glass with a controlled porosity or poly(styrene)benzene can achieve a faster flow rate and a shorter processing time than agarose. If the antibody comprises a CH3 domain, it can be purified using Bakerbond ABX. TM resin (J. T. Baker, Phillipsburg, N. J.). Other techniques for protein purification, such as fractionation in ion exchange columns, ethanol precipitation, reverse phase HPLC, silica gel chromatography, heparin agarose gel chromatography based on anion or cation exchange resin (e.g., polyaspartic acid columns), chromatofocusing, SDS-PAGE, and ammonium sulfate precipitation, may also be determined according to the antibody obtained as desired.

After any preliminary purification steps, the mixture containing the antibody of interest and impurities can be processed by a low pH hydrophobic interaction chromatography using an elution buffer with a pH of about 2.5-4.5, preferably at a low salt concentration (for example, from about 0 to 0.25M salt concentration).

### Kit

The application provides a kit containing the fusion protein. In some embodiments, the kit is used to detect the presence or level of OX40 in a biological sample. The biological sample may include cells or tissue.

In some embodiments, the kit contains a fusion protein conjugated to a detectable label. In some embodiments, the kit contains an unlabeled fusion protein, and further contains a labeled secondary antibody that can bind to the unlabeled fusion protein. The kit may further contain instructions for use and a package separating each component in the kit.

In some embodiments, the fusion protein is connected to a substrate or an instrument for a sandwich assay such as ELISA, or immunochromatographic assay. Suitable substrates or instruments may be, for example, microplates and test papers.

### Pharmaceutical composition and therapeutic method

The present application further provides a pharmaceutical composition comprising the fusion protein and one or more pharmaceutically acceptable carriers.

The pharmaceutically acceptable carriers for use in the pharmaceutical composition disclosed herein may include, for example, pharmaceutically acceptable liquids, gels, or solid carriers, aqueous media, non-aqueous media, antimicrobial materials, isotonic materials, buffers, antioxidants, anesthetics, suspending/dispersing agents, integrating agents, diluents, adjuvants, excipients, or nontoxic auxiliary substances, other components known in the art, or various combinations thereof.

Suitable components may include, for example, antioxidants, fillers, binders, disintegrating agents, buffers, preservatives, lubricants, flavoring agents, thickening agents, coloring agents, emulsifying agents, or stabilizing agents such as sugars and cyclodextrins. Suitable antioxidants may include, for example, methionine, ascorbic acid, EDTA, sodium thiosulfate, platinum, catalase, citric acid, cysteine, mercaptoglycerol, thioglycolic acid, mercaptosorbitol, butyl methylanisole, butylated hydroxytoluene, and/or propyl gallate. The inclusion of one or more antioxidants, such as methionine, in a composition comprising the fusion protein disclosed herein will reduce the oxidation of the fusion protein. A decreased oxidation may prevent or reduce the decrease in binding affinity, thereby improving antibody stability and extending shelf life.

Further, pharmaceutically acceptable carriers may include, for example, aqueous media such as sodium chloride injection, Ringer's solution injection, isotonic dextrose injection, sterile aqueous injection, or dextrose and lactated Ringer's solution, non-aqueous media such as plant-derived fixed oil, cottonseed oil, corn oil, sesame oil, or peanut oil, antibacterial substances at a bacteriostatic or fungistatic concentration, isotonic agents such as sodium chloride or dextrose, buffers such as phosphate or citrate buffers, antioxidants such as sodium bisulfate, local anesthetics such as procaine hydrochloride, suspending and dispersing agents such as sodium carboxymethylcellulose, hydroxypropylmethylcellulose or polyvinylpyrrolidone, emulsifying agents such as polysorbate 80 (Tween-80), integrating agents such as EDTA (ethylenediaminetetraacetic acid) or EGTA (ethyleneglycol bis(2-aminoethylether)tetraacetic acid), ethanol, polyethylene glycol, propylene glycol, sodium hydroxide, hydrochloric acid, citric acid or lactic acid. Antibacterial agents as the carrier can be added to a pharmaceutical composition in a multi-dose container, which include phenols or cresol, mercury formulations, benzyl alcohol, chlorobutanol, methyl and propyl para-hydroxybenzoate, merthiolate, benzalkonium chloride, and chlorophenethylamine. Suitable adjuvants may include, for example, water, salt, glucose, glycerol or ethanol. Suitable nontoxic auxiliary substances may include, for example, emulsifying agents, pH buffering agents, stabilizing agents, solubilizing agents, or substances such as sodium acetate, sorbitan laurate, triethanolamine oleate, or cyclodextrins.

The pharmaceutical composition may be liquid solution, suspension, emulsion, pill, capsule, tablet, sustained release formulation, or powder. Oral formulation may comprise standard carriers, such as pharmaceutical grade mannitol, lactose, starch, magnesium stearate, polyvinylpyrrolidone, sodium saccharin, cellulose, magnesium carbonate, and etc.

In some embodiments, the pharmaceutical composition is formulated as an injectable composition. The injectable pharmaceutical composition may be prepared into any conventional form, for example, liqiud solution, suspension, emulsion, or solid form suitable for production of liqiud soluiton, suspension, or emulsion. The injectable preparations may include ready-to-use sterile and/or pyrogen-free solution, sterile and dried soluble which is combined with a solvent immediately prior to use, such as lyophilized powder, and also include subcutaneous tablet, sterile suspension ready for injection, sterile and dried insoluble product which is combined with a vehicle immediately prior to use, and sterile and/or pyrogen-free emulsion. The solvent may be aqueous or non-aqueous.

In some embodiments, a unit dose of injectable preparation is packaged in an ampoule, a tube, or a syringe with a needle. It is known in the art that all formulations for injectable administration should be sterile and pyrogen-free.

In some embodiments, sterile lyophilized powders can be prepared by dissolving the antibody or antigen-binding fragment thereof disclosed herein in an appropriate solvent. The solvent may comprise one other pharmacological component that can increase the stability of the powder or a reconstituted solution prepared from the powder, or that improve the powder or the reconstituted solution. Suitable adjuvants include, but are not limited to, water, glucose, sorbitol, fructose, corn syrup, xylitol, glycerol, glucose, brown sugar, or other suitable substances. The solvent may comprise a buffer, such as citric acid buffer, sodium or potassium phosphate buffer, or other buffers known to those skilled in the art. In one embodiment, the pH of the buffer is neutral. The solution is then filtered and sterilized under standard conditions well known in the art, followed by lyophilization to produce a desired formulation. In one embodiment, the resulting formulation is subpackaged into vials and lyophilized. Each vial may contain a single dose or multiple doses of the anti-OX40 antibody or antigen-binding fragment thereof, or a combination thereof. The loading in each vial may be slightly higher than that required for each dose or multiple doses (e.g., 10% overdose) to ensure accurate sampling and administration. The lyophilized powder may be stored under suitable conditions, such as in a range of about 4°C to room temperature.

The lyophilized powder is re-dissolved with water for injection to obtain a formulation for administration by injection. In one embodiment, the lyophilized powder may be re-dissolved by being added into sterile and pyrogen-free water or other suitable liquid carrier. The precise amount is determined by the therapy chosen and may be determined with empirical value.

A method of treatment is also provided, comprising administering to a subject in need thereof a therapeutically effective amount of the fusion protein described herein.

The therapeutically effective dosage of the fusion protein provided herein will depend upon a variety of factors well known in the art, such as body weight, age, past medical history, current therapy, the health of the subject and the potential for cross-infection, allergies, hypersensitivity, and side effects, as well as the route of administration and the extent of tumor development. Those skilled in the art (e.g., physicians or veterinarians) can proportionally reduce or increase the dosage according to these or other conditions or requirements.

In some embodiments, the fusion protein provided herein may be administered at a therapeutically effective dose of between about 0.01 mg/kg and about 100 mg/kg. In some embodiments, the fusion protein is administered at a dose of about 50 mg/kg or less, and in some embodiments at a dose of 10 mg/kg or less, 5 mg/kg or less, 1 mg/kg or less, 0.5 mg/kg or less, or 0.1 mg/kg or less. A particular dose may be administered at multiple intervals, for example, once a day, twice or more a day, twice or more a month, once a week, once every two weeks, once every three weeks, once a month, or once every two months or more months. In some embodiments, the dosage administered may vary with the course of the treatment. For example, in some embodiments, the initial dose administered may be higher than the subsequent dose administered. In some embodiments, the dosage administered is adjusted over the course of treatment according to the response of the subject being administered.

The dosage regimen may be adjusted to achieve an optimal response (e.g., a therapeutic response). For example, administration may be conducted in a single dose or in multiple divided doses over two periods of time.

The fusion protein disclosed in the present invention may be administered by the administration modes well-known in the art, for example, by injection (e.g., subcutaneous injection, intraperitoneal injection, intravenous injection including intravenous drip, intramuscular injection, or intradermal injection) or non-injection (e.g., oral administration, nasal administration, sublingual administration, rectal administration, or topical administration).

In some embodiments, the fusion protein is used for treating conditions associated with its molecular mechanisms, including tumors and cancers, for example, non-small cell lung cancer, small cell lung cancer, renal cell cancer, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic carcinoma, leukemia, lymphoma, myeloma, mycoses fungoids, Merkel-cell carcinoma and other hematological malignancies such as classical Hodgkin's lymphoma (CHL), primary mediastinal large B-cell lymphoma, T-cell/histiocyte B-cell-rich lymphoma, EBV positive and negative PTLD and EBV-associated diffuse large B-cell lymphoma (DLBCL), plasmablastic lymphoma, extranodal NK/T-cell lymphoma, nasopharyngeal carcinoma and HHV 8-associated primary exudative lymphoma, Hodgkin's lymphoma, central nervous system (CNS) tumors such as primary CNS lymphoma, spinal tumors, brain stem glioma. In some embodiments, the conditions treated by the fusion protein include chronic viral infections, such as viral infection of hepatitis B virus, hepatitis C virus, herpes virus, Epstein-Barr virus, HIV, cytomegalovirus, herpes simplex virus type I, herpes simplex virus type 2, human papilloma virus, or adenovirus, Kaposi's sarcoma-associated herpesvirus epidemics, and infection of Torquetenovirus, JC virus, or BK virus.

### Method of application

The present application further provides a method of using the fusion protein.

In some embodiments, the present application provides a method of treating diseases or conditions associated with the mechanisms of the fusion protein in an individual, comprising administering a therapeutically effective amount of the fusion protein described herein.

The fusion protein disclosed herein may be administered alone or in combination with one or more other therapeutic means or agents. For example, the fusion protein disclosed herein may be used in combination with chemotherapy, radiation therapy, cancer treatment surgery (e.g., tumor resection), an anti-viral drug, one or more anti-emetic agents or other therapies for chemotherapy-induced complications, or any other therapeutic agent for cancers or viruses. In some such embodiments, the fusion protein disclosed herein, when used in combination with one or more therapeutic agents, may be administered simultaneously with the one or more therapeutic agents, and in some such embodiments, the fusion protein may be administered simultaneously as a part of the same pharmaceutical composition. However, the fusion protein "in combination" with other therapeutic agent need not be administered simultaneously or in the same composition comprising the therapeutic agent. The meaning of "in combination" in the present invention also includes that a fusion protein administered before or after another therapeutic agent, which is also considered to be "in combination" with the therapeutic agent, even if the fusion protein and the second agent are administered by different administration modes. Where possible, other therapeutic agent for use in combination with the fusion protein disclosed herein may be administered according to the methods described in the manufacturer's instructions for such other therapeutic agent, or according to Physicians' Desk Reference 2003 (Physicians' Desk Reference, 57th Ed; Medical Economics Company; ISBN: 1563634457; 57th Ed (2002.11)), or according to other methods known in the art.

In some embodiments, the therapeutic agent is capable of inducing or enhancing an immune response to cancers. For example, tumor vaccines may be used to induce an immune response to certain tumors or cancers. Cytokine therapy may be used to enhance the presentation of tumor antigen to the immune system. Examples of cytokine therapy include, but are not limited to, interferons such as interferon α, β and γ, colony stimulating factors such as macrophage CSF, granulocyte macrophage CSF and granulocyte CSF, interleukins such as IL-L, IL-1a, IL-2, IL-3, IL-4, IL-5, IL-6, IL-7, IL-8, IL-9, IL-10, IL-11, and IL-12, tumor necrosis factors such as TNF-α and TNF-β. Agents that inactivate immunosuppressive targets, such as PD-L1/PD-1 antibodies, TGF-β inhibitors, IL-10 inhibitors, and Fas ligand inhibitors, may also be used. Another group of agents include those that activate an immune response against tumor or cancer cells, for example, those that improve T cell activation (e.g., T cell co-stimulatory molecule agonists such as CTLA-4, ICOS), and those that increase dendritic cell functions and antigen presentation.

The following examples are intended to better illustrate the present invention, and should not be construed as limiting the scope of the present invention. All of the specific compositions, materials, and methods described below, as a whole or a part, are within the scope of the present invention. These specific compositions, materials, and methods are not intended to limit the present invention, but are merely illustrative of the specific embodiments within the scope of the present invention. Equivalent compositions, materials and methods may be developed by those skilled in the art without addition of inventive effort and without departing from the scope of the present invention. It will be appreciated that various modifications made to the method of the present invention may still fall within the scope of the present invention. The inventors intend to include such variations within the scope of the present invention.

### Example 1: Preparation of the fusion protein of the present invention

This example illustrates the design and expression of several anti-OX40 antibody-human interferon fusion proteins, wherein the heavy and light chain sequences of the anti-human OX40 activating antibodies were derived from MT01-C1 or MT01-C1 (G2) in the Chinese Patent 201711476160.3, wherein "MT01-C1" refers to a monoclonal antibody having the same VH (SEQ ID NO: 7) and VL (SEQ ID NO: 8) sequences as UMY02-L1, UMY02-L2 and UMY02-L3, the heavy and light chain constant regions of which are human IgG1 and κ chain, respectively. "MT01-C1(G2)" refers to a monoclonal antibody having the same VH (SEQ ID NO: 7) and VL (SEQ ID NO: 8) sequences as UMY02-L1, UMY02-L2 and UMY02-L3, the heavy and light chain constant regions of which are human IgG2 and κ chain, respectively.

The interferon IFNα2b sequence was taken from the human interferon IFNα2b (NP_000596.2), the amino acid sequence of which is as shown in SEQ ID NO: 9.

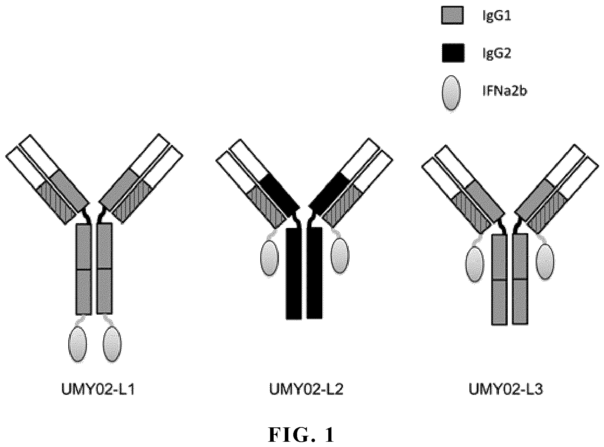

The structural design of exemplary fusion proteins are as shown in FIG. 1. In FIG. 1, "UMY02-L1" refers to a fusion protein having the heavy chain, the peptide linker and the human interferon as shown in SEQ ID NO. 10, and the light chain as shown in SEQ ID NO. 11, wherein the human interferon IFNα2b as shown in SEQ ID NO. 9 is linked to the carboxyl-terminus of the heavy chain by the peptide linker; "UMY02-L2" refers to a fusion protein having the heavy chain as shown in SEQ ID NO. 12, and the light chain, the peptide linker and the human interferon as shown in SEQ ID NO. 13, wherein the human interferon IFNα2b as shown in SEQ ID NO. 9 is linked to the carboxyl-terminus of the light chain by the peptide linker; "UMY02-L3" refers to a fusion protein having the heavy chain as shown in SEQ ID NO. 14, and the light chain, the peptide linker and the human interferon as shown in SEQ ID NO. 13, wherein the human interferon IFNα2b as shown in SEQ ID NO. 9 is linked to the carboxyl-terminus of the light chain via the peptide linker.

The cDNA sequences encoding the heavy and light chains of the antibodies of the fusion proteins were cloned into the mammalian cell expression vector pcDNA 3.4, respectively. The heavy chain expression plasmids and light chain expression plasmids were transfected into HEK293 cells with Lipofectamine 2000 transfection reagent (Invitrogen) at a molar ratio of 2:1, and cultured for 7 days at 37°C and 5% carbon dioxide. The supernatants were collected, and the antibodies in the supernatants were purified by Protein A affinity chromatography. The purified antibodies were dialyzed against PBS solution, concentrated by lyophilization and stored at -20°C.

### Example 2: ELISA binding assay

A 96-well high affinity plate was coated with 1 (µg/mL human OX40 protein solution at 100 µL/well and shaken overnight at 4°C. The next day, the plate was first washed 3 times with 300 µL PBST (Tween 20: 0.5 ‰), then blocked with 5% BSA/PBS at 100 µL/well for 2 h, and shaken at room temperature, followed by washing 3 times with 300 µL PBST. Gradient dilutions of the fusion protein samples were prepared in PBS, and then added to the 96-well plate at 100 µL/well; the plate was shaken for 1 h at room temperature, followed by washing 3 times with 300 µL PBST. A secondary antibody goat-anti-human IgG HRP solution was prepared and added to the 96-well plate at 100 µL/well, and the plate was shaken for 1 h at room temperature, followed by washing 4 times with 300 µL PBST. TMB was added at 100 µL/well to develop color for 20 min. 0.6N H₂SO₄ was added at 100 (µL/well to stop the color development, and the OD values were determined at 450 nm.

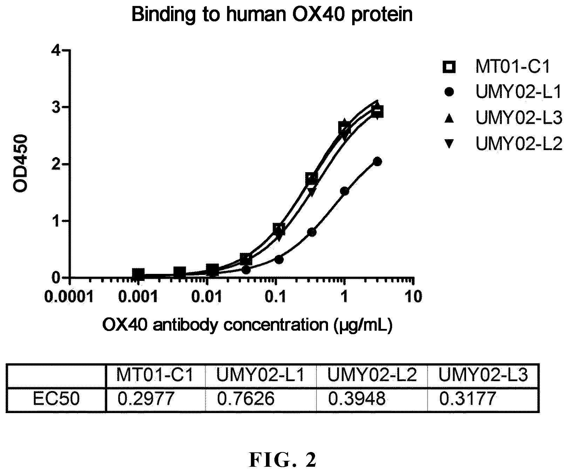

After testing, the results are shown in FIG. 2. The EC50 of the fusion proteins UMY02-L1, UMY02-L2, and UMY02-L3 for ELISA binding were 0.7626, 0.3948, and 0.3177 µg/mL, respectively, all of which were equivalent to EC50 (0.2977 µg/mL) of the OX40 antibody MT01-C1 (FIG. 2).

### Example 3: Binding to human, cvnomolgus monkey and mouse OX40 (FACS)

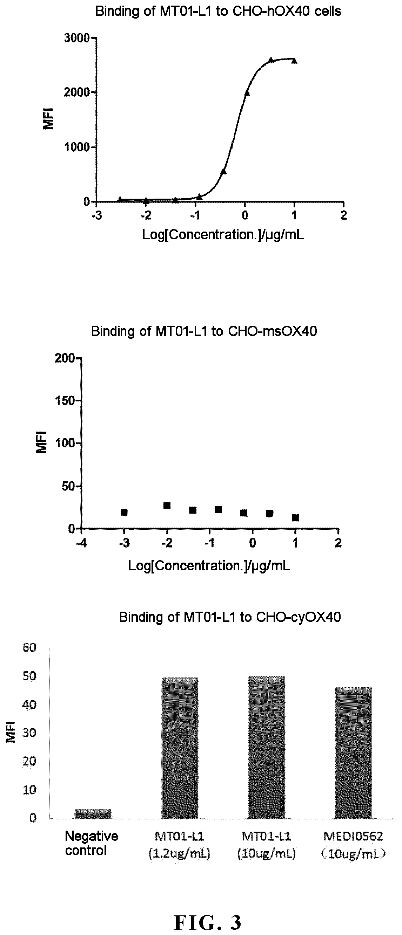

CHO cells were transfected with an expression plasmid encoding human, cynomolgus or mouse OX40 protein and cultured for 48 h. The solutions of the OX40 antibody MT01-L1 having a concentration gradient were prepared with PBS, each of which being a working solution of 10× final concentration. CHO-hOX40 cells were harvested, washed once with PBS, followed by counting and diluting to 2×10⁶/ml cell suspension; 10 µL the OX40 antibody MT01-L1 working solutions were added to 100 µL the cell suspension, respectively, followed by incubating at 4°C for 30 min in the dark; after washing twice with PBS, a secondary antibody was added, followed by incubating at 4°C for 30 min in the dark; after washing once with PBS, the resultants were suspended in 400 µL FACS buffer and tested on the machine. As shown in FIG. 3, the results show that MT01-L1 bound to human OX40 (hsOX40) with an EC50 of 0.66 (µg/mL.

Similarly, the present inventors tested the binding of the antibody to CHO cells expressing mouse OX40, and found that MT01-L1 did not bind to mouse OX40 (msOX40).

Similarly, the present inventors tested the binding of the antibody to CHO cells expressing cynomolgus monkey OX40, and found that MT01-L1 bound to cynomolgus monkey OX40 (cyOX40) significantly.

### Example 4: Daudi cell proliferation inhibition assay

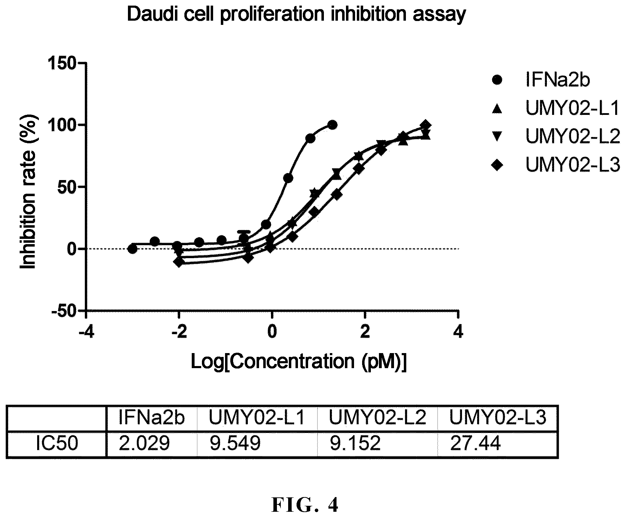

Interferon receptors are highly expressed on Daudi cells (ATCC), so interferon has a biological activity on the cells. Daudi cells were plated into a 96-well plate at 20,000 cells/90 µL/well; the samples to be tested were prepared into 10 × working solutions by being diluted according to the concentration gradient, and added into the 96-well plate at 10 µL/well and placed in a 37°C incubator, respectively; after 72 h, the OD450 values were determined by adding CCK8 and the proliferation inhibition rate was calculated for the cells in each well. This inhibition rate reflects the activity of interferon in the samples. The experimental results show that the proliferation inhibitory activities (IC50) of the fusion proteins UMY02-L1, UMY02-L2 and UMY02-L3 on Daudi cells were 9.549, 9.152 and 27.44 pM, respectively (FIG. 4).

### Example 5: FcR-mediated OX40 signaling pathway activation assay

The present inventors constructed a cellular assay system for detecting OX40 activators. Specifically, the present inventors constructed cell strains stably transfected by "Jurkat-OX40-NFκB-luciferase reporter gene (Luc)", which could activate the expression of NFκB-luciferase reporter gene when the OX40 activating antibody was mixed with the stably transfected cell strains and HEK293 cells overexpressing FcR.

The solutions of the fusion proteins having a concentration gradient were prepared with PBS on ice, each of which being a working solution of 2 × final concentration. Jurkat-NFκB-luc-OX40 cells and HEK293 cells overexpressing FcR were harvested, centrifuged, then resuspended in the culture medium, and plated into a 384-well plate. The fusion protein working solutions and a proper amount of cell suspension were added into the 384-well plate. After 5 h of incubation, One-Glo (Promega) detection reagent was added, and after uniform mixing the fluorescence signals were detected by Pherastar autofocus fluorescence microplate reader.

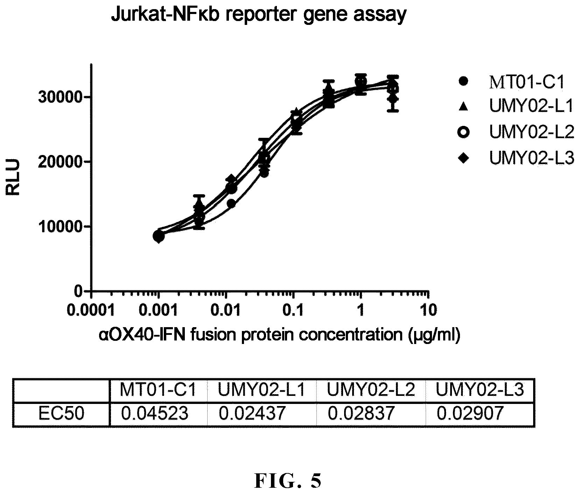

As shown in FIG. 5, the OX40 antibody MT01-C1 and the fusion proteins UMY02-L1, UMY02-L2 and UMY02-L3 were detected to activate NFκB-luciferase reporter with the EC50 of 0.04523, 0.02437, 0.02837 and 0.02907 ng/mL, respectively, in the above experimental system. The results show that FcR-mediated OX40 activation activities of UMY02-L1, UMY02-L2 and UMY02-L3 were stronger than that of MT01-Cl.

### Example 6: Interferon receptor-mediated OX40 signaling pathway activation assay

The cell strains transfected stably by "Jurkat-OX40-NFκB-luciferase reporter" were plated into a 384-well plate at 10,000/well. The solutions of the fusion protein having a concentration gradient and a control group solution were prepared with PBS on ice, each of which being a working solution of 2 × final concentration. The fusion protein or the interferon working solution and a proper amount of cell suspension were added into the 384-well plate. After 5 h incubation, One-Glo (Promega) detection reagent was added, and after uniform mixing the fluorescence signals were detected by Pherastar autofocus fluorescence microplate Reader.

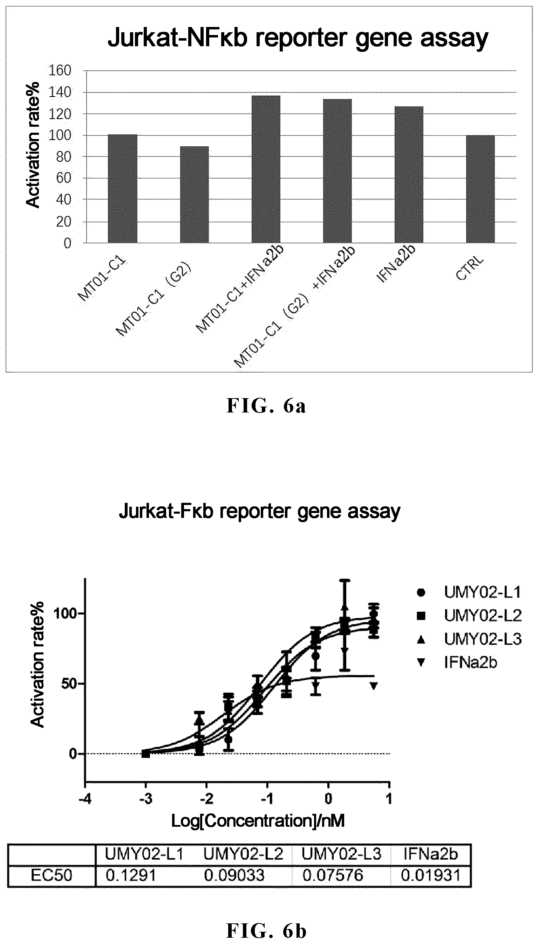

As shown in FIG. 6a, the final concentrations of all antibodies or the interferon in the assay were 10 nM. The OX40 antibody MT01-C1 or MT01-C1 (G2) did not show the activating effect on the OX40 signaling pathway in Jurkat cells, and addition of interferon IFNα2b alone or simultaneous addition of the OX40 antibody MT01-C1 and IFNα2b had an activation effect of less than 40% compared to the control group. However, when the fusion protein UMY02-L1, UMY02-L2 or UMY02-L3 was added, the OX40 signaling pathway in Jurkat cells was significantly activated, and the activation degree was significantly greater than that of the interferon group alone (FIG. 6b).

### Example 7: Pharmacokinetics assay in C57BL/6 Mice

Eighteen female C57BL/6 mice aged 6-8 weeks were divided into 3 groups, 6 for each group, and were injected intravenously with UMY02-L1, UMY02-L3, and MT01-C1 respectively. The dose administered was 5 mg/kg. For UMY02-L1 or UMY02-L3, the peripheral venous blood was collected from the animals before the administration and at 1, 2, 6, 24, 48, 72, 96, 174, 220 and 288 h after the administration; for MT01-C1, the peripheral venous blood was collected from the animals before the administration and at 1, 2, 6, 24, 48, 72, 96, 192 and 312 h after the administration; the serums were collected by centrifugation. The serums were collected from 3 animals at each time point, and the blood samples were collected alternately from 6 animals in each group at different time points.

A 96-well high affinity plate was coated with 1 (µg/mL human OX40 protein solution at 100 µL/well and shaken overnight at 4°C. The next day, the plate was first washed 3 times with 300 µL PBST (Tween 20: 0.5 ‰), then blocked with 5% BSA/PBS at 100 µL/well for 1 h, and shaken at room temperature, followed by washing 4 times with 300 µL PBST. 100-fold diluted solutions of serum samples to be tested and control serum solutions with different concentrations were prepared by PBS, and then added to the 96-well plate at 100 µL/well; the plate was shaken for 1.5 h at room temperature, followed by washing 4 times with 300 µL PBST. For MT01-C1, a solution of secondary antibody donkey-anti-human IgG HRP (Jackson ImmunoResearch, Art. No. 709-035-149) was prepared and added into the 96-well plate at 100 µL/well, and the plate was shaken for 1 h at room temperature, followed by washing 4 times with 300 µL PBST. TMB was added at 100 µL/well to develop color for 20 min. 0.6N H₂SO₄ was added at 100 µL/well to stop the color development, and the OD values were determined at 450 nm. For UMY02-L1 and UMY02-L3, 0.5 µg/mL rabbit-anti-human IFN (Abcam, Art. No. ab222552) solution was prepared and added to the 96-well plate at 100 µL/well, and the plate was shaken for 1.5 h at room temperature, followed by washing 4 times with 300 µL PBST. A secondary antibody goat-anti-rabbit IgG HRP (GenScript Biotech Corp., Art. No. A00098) solution was prepared and added to the 96-well plate at 100 µL/well. and the plate was shaken for 1 h at room temperature, followed by washing 4 times with 300 µL PBST. 100 µL/well TMB was added to develop color for 20 min. 0.6N H₂SO₄ was added at 100 µL/well to stop the color development, and the OD values were determined at 450 nm. Logistic four-parameter fitting was conducted on the detection values of control solutions with different concentrations relative to the concentrations of control solutions to obtain a standard curve and a regression equation. The detected values of the samples to be tested were substituted into the equation for calculation to obtain the serum drug concentrations at different time points.

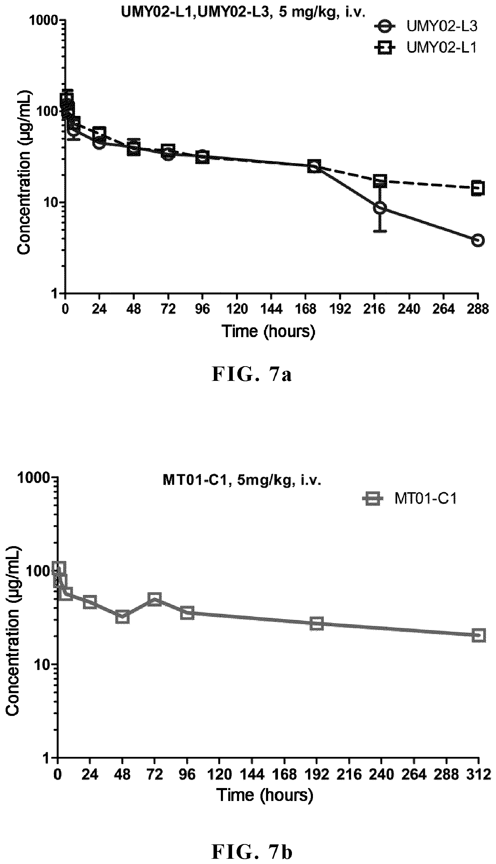

As shown in FIGs. 7a and 7b, the mice in the experiment were injected with 5 mg/kg UMY02-L3 or UMY02-L1 intravenously. 7 days after the administration, the serum drug concentration of UMY02-L3 was still above 10 µg/mL, and 12 days after the administration, the serum drug concentration of UMY02-L1 was still above 10 (µg/mL. indicating the fusion proteins UMY02-L3 and UMY02-L1 have the pharmacokinetic characteristics similar to the antibody drug MT01-C1 in mice, and a much longer half-life than 2-3 h of IFNα2b (see product manual for Intron A, Merck).

### Example 8: Effect of antibody subtype and peptide linker length on the activity of antibody fusion protein

To investigate the effects of length of a peptide linker linking an OX40 activating antibody and interferon IFNα2b, and antibody subtype on OX40 signaling pathway activating activity of the fusion proteins, the antibody fusion proteins with peptide linkers having different lengths and different antibody subtypes were constructed based on UMY02-L3 (see Table 1).

The heavy chains of the fusion proteins UMY02-L4, UMY02-L5 and UMY02-L6 are as shown in SEQ ID NO: 14, and the light chain and the human interferon are as shown in SEQ ID NO: 13, with the only difference in the number of peptide linker, as shown in Table 1 below.

The heavy chains of the fusion proteins UMY02-L7 and UMY02-L8 are as shown in SEQ ID NO: 15, and the light chain and the human interferon are as shown in SEQ ID NO: 13, with the only difference in the number of peptide linker, as shown in Table 1 below.

The effects of these antibody fusion proteins on Daudi cell proliferation were tested according to the method in Example 4, and as a result it was found that different peptide linker lengths have a significant effect on the interferon activity of the antibody fusion proteins, namely, the shorter the peptide linker length is, the lower the interferon activity is (see Table 1).

The activities of these fusion proteins for activating the OX40 signaling pathway were tested using the method in Example 5. As shown in Table 1, the OX40 antibody MT01-C1 and the fusion proteins of various antibodies were detected to be effective in activating the OX40 signaling pathway in Jurkat-OX40 cells.

**Table 1 Effects of antibody subtype and linker length on interferon activity**

| Name | Antibody subtype | Peptide linker | **Daudi cell proliferation assay** | | **Activation activity of OX40 signaling pathway** | |
|---|---|---|---|---|---|---|
| | | | IC50 (pM) | Relative activity | EC50 (nM) | Relative activity |
| MT01-C1 | IgG1 | / | / | / | 0.178 | 1.00 |
| IFNα2b | / | / | 0.7982 | 1.00 | | |
| UMY02-L3 | IgG1 | (GGGGS)3 | 21.45 | 3.72E-02 | 0.179 | 0.99 |
| UMY02-L4 | IgG1 | Without a peptide linker | 855.3 | 9.33E-04 | 0.280 | 0.64 |
| UMY02-L5 | IgG1 | GGGGS | 86.75 | 9.20E-03 | 0.302 | 0.59 |
| UMY02-L6 | IgG1 | (GGGGS)2 | 53.20 | 1.50E-02 | 0.309 | 0.58 |
| UMY02-L7 | IgG4 | GGGGS | 1263.00 | 6.32E-04 | 0.497 | 0.36 |
| UMY02-L8 | IgG4 | (GGGGS)2 | 259.1 | 3.08E-03 | 0.358 | 0.50 |

### Example 9: Fusion of OX40 antibody to different IFNα2b mutant proteins

The OX40 activating antibody may also be fused to an interferon mutant. The specific activities of the mutant interferons are lower than those of wild-type interferons, so that the mutant interferons may act synergistically with the OX40 antibody at the same drug concentration, and meanwhile the toxic and side effects caused by excessive interferon activity are avoided.

In this example, we constructed and expressed a series of OX40 antibody-mutant interferon fusion proteins using the HEK 293 expression system (Table 2). In these antibody fusion proteins, the antibody subtype of OX40 was IgG 4, while the IFNα2b portion carried a T106A mutation to remove its glycosylation site.

The heavy chains of the fusion proteins UMY02-L13, UMY02-L14, UMY02-L15, UMY02-L16, UMY02-L17 and UMY02-L18 are as shown in SEQ ID NO: 15, and the light chain and the human interferon are as shown in SEQ ID NO: 13, only with the following differences:
1. The peptide linkers of UMY02-L13, UMY02-L14, UMY02-L15, UMY02-L16 and UMY02-L18 are (GGGGS)2, and the peptide linker of UMY02-L17 is (GGGGS)3;
2. The interferons of the fusion proteins UMY02-L13, UMY02-L14, UMY02-L15, UMY02-L16, UMY02-L17 and UMY02-L18 have the mutattion as shown in Table 2 relative to SEQ ID NO: 9.

The effects of these antibody fusion proteins on Daudi cell proliferation were tested according to the method in Example 4, and as a result it was found that the interferon activities of the mutant antibody fusion proteins were significantly lower than that of the wild-type IFNα2b (Table 2). Meanwhile, the activation effects of OX40 signaling pathway in Jurkat cells by these antibody fusion proteins were tested according to the method in Example 5, and the results show that the OX40 activation activities of these mutant antibody fusion proteins were substantially unchanged (Table 2). This result indicats that a suitable OX40 antibody-IFN fusion protein could be obtained by introducing a specific mutation into the interferon sequence, ensuring that the OX40 activating activity and the interferon activity are matched at an equal molar concentration, exert their functions synergistically in human body, and avoid a poor therapeutic effect or severe toxic and side effects caused by too high or low activity of one of them.

**Table 2 Activities of mutated antibody fusion proteins**

| | | **Daudi cell proliferation assay** | | **Activation activity of OX40 signaling pathway** | |
|---|---|---|---|---|---|
| | Interferon mutation ¹ | IC50 (pM) | Relative activity ² | EC50 (nM) | Relative activity ³ |
| MT01-C1 | / | / | / | 0.3251 | 1.00 |
| IFNα2b | wt | 0.683 | 1.00 | / | / |
| UMY02-L13 | T106A/A145D | 28589 | 2.39E-05 | 0.4972 | 0.65 |
| UMY02-L 14 | T106A/R149A | 18110 | 3.77E-05 | 0.4634 | 0.70 |
| UMY02-L15 | T106A/R120A | 1892 | 3.61E-04 | / | / |
| UMY02-L 16 | T106A/A145G | 2069 | 3.30E-04 | 0.5661 | 0.57 |
| UMY02-L 17 | T106A/R149A | 16094 | 4.24E-05 | 0.4608 | 0.71 |
| UMY02-L 18 | T106A/L117A | 3318 | 2.06E-04 | 0.5410 | 0.60 |

| | | | | | |
|---|---|---|---|---|---|
| 1. The position number of mutated amino acid is indicated referring to the amino acid sequence of wild-type human interferon IFNα2b, SEQ ID NO: 9. 2. The activity of IFNα2b is defined as 1. 3. The activity of the OX40 mAb MT01-C1 is defined as 1. | | | | | |

### Example 10 Killing effects of the fusion proteins on tumors cell

Further, the inventors fused the heavy chain of the OX40 antibody to different types of interferons to produce different fusion proteins, wherein the heavy chains of OX40 IFN-α2a, OX40-IFNβ, OX40-IFNy, and OX40-IFNγ3 are as shown in SEQ ID NO: 15; the light chain, the peptide linker and the interferon of the OX40 IFN-α2a are as shown in SEQ ID NO: 16; the light chain, the peptide linker and the interferon of the OX40 IFN-β are as shown in SEQ ID NO: 17; the light chain, the peptide linker and the interferon of the OX40 IFN-γ are as shown in SEQ ID NO: 18; and the light chain, the peptide linker and the interferon of OX40 IFN-λ3 are as shown in SEQ ID NO: 19.

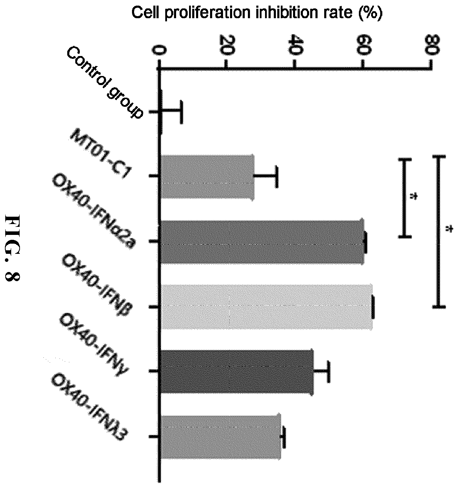

The killing effects of these antibody fusion proteins on tumor cell were tested. Specifically, the inventors mixed and co-cultured human-derived PBMCs and ovarian cancer SKOV3 cells at a ratio of 20: 1, and stimulated them with PBS (control), or the OX40 mAb MT01-C1 and the different antibody fusion proteins (OX40IFN-α2a, OX40-IFNβ, OX40-IFNγ, OPX40-1FNλ3 at a concentration of 0.1 nM, respectively; after 48 h of cultivation at 37°C, 5% CO₂, the cytotoxicities of PBMCs on SKOV3 cells were detected by CCK8 kit. The experimental results are shown in FIG. 8, showing that PBMCs had no an inhibitory effect on SKOV3 cell proliferation without stimulation; the inhibition rate of PBMCs on SKOV3 was about 28% under the stimulation by the OX40 mAb; however, under the stimulation of fusion proteins, the cytotoxicities of PBMC were stronger than that under the stimulation of the OX40 mAb, wherein the inhibition rates under the stimulation of the OX40 mAb MT01-Cl-IFNα2a and the OX40 mAb MT01-C1-IFNβ were significantly enhanced, i.e., 60% and 63%, respectively.

The above are merely the preferred examples of the present invention, which do not impose any limitation on the present invention. Any form of equivalent replacement or modification and the like performed by those skilled in the art on the technical solutions and technical contents disclosed by the present invention without departing from the scope of the technical solutions of the present invention belong to the contents that do not deviate from the technical solutions of the present invention and still fall within the protection scope of the present invention.

Main References:
1. Willoughby J, Griffiths J, Tews I, Cragg MS. OX40: Structure and function - What questions remain? Mol Immunol. 2017; 83: 13-22. doi: 10.1016/j.molimm.2017.01.006.
2. Imura A, Hori T, Imada K, Ishikawa T, Tanaka Y, Maeda M, Imamura S, Uchiyama T. The human OX40/gp34 system directly mediates adhesion of activated T cells to vascular endothelial cells. J Exp Med. 1996; 183: 2185-95. doi:
3. Burgess JK, Carlin S, Pack RA, Arndt GM, Au WW, Johnson PR, Black JL, Hunt NH. Detection and characterization of OX40 ligand expression in human airway smooth muscle cells: a possible role in asthma? J Allergy Clin Immunol. 2004; 113: 683-9. doi: 10.1016/j .jaci.2003.12.311.
4. Compaan DM, Hymowitz SG. The crystal structure of the costimulatory OX40-OX40L complex. Structure. 2006; 14: 1321-30. doi: 10.1016/j.str.2006.06.015.
5. Song J, So T, Croft M. Activation of NF-kappaB1 by OX40 contributes to antigen-driven T cell expansion and survival. J Immunol. 2008; 180: 7240-8. doi:
6. Croft M. Control of immunity by the TNFR-related molecule OX40 (CD134) . Annu Rev Immunol. 2010; 28: 57-78. doi: 10.1146/annurev-immunol-030409-101243.
7. Rogers PR, Song J, Gramaglia I, Killeen N, Croft M. OX40 promotes Bcl-xL and Bcl-2 expression and is essential for long-term survival of CD4 T cells. Immunity. 2001; 15: 445-55. doi:
8. Song J, So T, Cheng M, Tang X, Croft M. Sustained survivin expression from OX40 costimulatory signals drives T cell clonal expansion. Immunity. 2005; 22: 621-31. doi: 10.1 016/j .immuni.2005.03.0 12.
9. So T, Song J, Sugie K, Altman A, Croft M. Signals from OX40 regulate nuclear factor of activated T cells cl and T cell helper 2 lineage commitment. Proc Natl Acad Sci USA. 2006; 103: 3740-5. doi: 10.1073/pnas.0600205103.
10. Publicover J, Gaggar A, Jespersen JM, Halac U, Johnson AJ, Goodsell A, Avanesyan L, Nishimura SL, Holdorf M, Mansfield KG, Judge JB, Koshti A, Croft M, et al. An OX40/OX40L interaction directs successful immunity to hepatitis B virus. Sci Transl Med. 2018; 10. doi: 10. 1126/scitranslmed.aah5766.

## Claims

1. A fusion protein comprising:
a) an antibody or antigen-binding fragment thereof that specifically binds to human OX40; and
b) a human interferon; wherein the human interferon is linked to the carboxyl- or amino-terminus of the light or heavy chain of the antibody directly or via a peptide linker.

2. The fusion protein according to claim 1, wherein the antibody or antigen-binding fragment thereof that specifically binds to human OX40 comprises:
an antibody heavy chain variable region comprising VH CDR1 having the amino acid sequence of SEQ ID NO: 1, VH CDR2 having the amino acid sequence of SEQ ID NO: 2, and VH CDR3 having the amino acid sequence of SEQ ID NO: 3; and
an antibody light chain variable region comprising VL CDR1 having the amino acid sequence of SEQ ID NO: 4, VL CDR2 having the amino acid sequence of SEQ ID NO: 5, and VL CDR3 having the amino acid sequence of SEQ ID NO: 6.

3. The fusion protein according to claim 2, wherein the heavy chain variable region comprises the amino acid sequence as shown in SEQ ID NO: 7, and the light chain variable region comprises the amino acid sequence as shown in SEQ ID NO: 8.

4. The fusion protein according to any one of claims 1 to 3, wherein the antibody or antigen-binding fragment thereof that specifically binds to human OX40 is a camelized single-domain antibody, scFv, a scFv dimer, BsFv, dsFv, dsFv2, dsFv-dsFv', a Fv fragment, Fab, Fab', F(ab')2, a ds diabody, a nanobody, a domain antibody, or a bivalent domain antibody.

5. The fusion protein according to any one of claims 1 to 4, wherein the antibody further comprises a constant region of immunoglobulin; preferably, the constant region is a constant region of human IgG1, IgG2 or IgG4.

6. The fusion protein according to any one of claims 1 to 5, wherein the human interferon is selected from the group consisting of human interferon type I, human interferon type II, and human interferon type III; preferably, the human interferon is IFNα2a, IFNβ, IFNγ, IFNλ3, or IFNα2b; more preferably, the human interferon is IFNα2b having the amino acid sequence as shown in SEQ ID NO: 9; further preferably, the human interferon is an IFNα2b mutant, which has one or more mutations selected from the group consisting of: T106A, R149A, A145G, A145D, R120A, and L117A relative to the amino acid sequence as shown in SEQ ID NO: 9; further more preferably, the IFNα2b mutant has one or more double mutations selected from the group consisting of: T106A/A145D, T106A/R149A, T106A/A145G, T106A/R120A, and T106A/L117A relative to the amino acid sequence as shown in SEQ ID NO: 9.

7. The fusion protein according to any one of claims 1 to 7, wherein the peptide linker is selected from the group consising of (G)ₙ, KESGSVSSEQLAQFRSLD, EGKSSGSGSESKST, GSAGSAAGSGEF, (GGGGS)ₙ, and (GGSGG)ₙ; preferably, the peptide linker is (GGGGS)ₙ; wherein n is an integer between 0 and 5; preferably, n is an integer between 1 and 3.

8. The fusion protein according to any one of claims 1 to 7, wherein the fusion protein is one or more selected from the group consisting of: UMY02-L1, UMY02-L2, UMY02-L3, UMY02-L4, UMY02-L5, UMY02-L6, UMY02-L7, UMY02-L8, UMY02-L13, UMY02-L14, UMY02-L15, UMY02-L16, UMY02-L17, UMY02-L18, OX40 IFN-α2a, OPX40-1FNβ OX40-IFNγ, and OX40-1FNλ3

9. An isolated polynucleotide encoding the fusion protein according to any one of claims 1 to 8.

10. A vector comprising the isolated polynucleotide according to claim 9.

11. A host cell comprising the vector according to claim 10.

12. A method of expressing the fusion protein according any one of claims 1 to 8, comprising culturing the host cell according to claim 11 under conditions capable of expressing the isolated polynucleotide according to claim 9.

13. A kit comprising the fusion protein according to any one of claims 1 to 8.

14. A pharmaceutical composition comprising the fusion protein according to any one of claims 1 to 8 and a pharmaceutically acceptable carrier.

15. Use of the fusion protein according to any one of claims 1 to 8 in preparing a medicament for treating a condition benefiting from enhancing an immune response and/or exposure to an interferon; preferably, the condition is cancer or viral infection; more preferably, the viral infection is hepatitis B virus infection.

16. A method of treating a condition benefiting from enhancing an immune response and/or exposure to an interferon, comprising administering to a subject in need thereof a therapeutically effective amount of the fusion protein according to any one of claims 1 to 8 or the pharmaceutical composition according to claim 14; preferably, wherein the condition is cancer or viral infection; more preferably, wherein the viral infection is hepatitis B virus infection.


SEQUENCE LISTING

<110>  NANJING UMAB-BIOPHARMA CO., LTD.

<120>  FUSION PROTEIN AND ITS APPLICATON IN PREPARING MEDICINE FOR TREATING
TUMOR AND/OR VIRAL INFECTION

<130>  EIC19310016P

<160>  19

<170>  SIPOSequenceListing 1.0

<210>  1
<211>  10
<212>  PRT
<213>  Artificial Sequence

<400>  1
Gly Tyr Thr Phe Thr Ser Tyr Val Met His
1               5                   10

<210>  2
<211>  19
<212>  PRT
<213>  Artificial Sequence

<400>  2
Tyr Ile Asn Pro Tyr Asn Asp Gly Thr Lys Tyr Asn Glu Lys Phe Lys
1               5                   10                  15
Gly Lys Ala

<210>  3
<211>  12
<212>  PRT
<213>  Artificial Sequence

<400>  3
Ala Asn Tyr Tyr Gly Ser Lys Phe Ala Met Asp Tyr
1               5                   10

<210>  4
<211>  11
<212>  PRT
<213>  Artificial Sequence

<400>  4
Arg Ala Ser Gln Asp Ile Ser Asn Tyr Leu Ser
1               5                   10

<210>  5
<211>  7
<212>  PRT
<213>  Artificial Sequence

<400>  5
Tyr Thr Ser Arg Leu His Ser
1               5

<210>  6
<211>  9
<212>  PRT

<213>  Artificial Sequence

<400>  6
Gln Gln Gly Ile Thr Leu Pro Trp Thr
1               5

<210>  7
<211>  119
<212>  PRT
<213>  Artificial Sequence

<400>  7
Glu Val Gln Leu Val Gln Ser Gly Ala Glu Val Lys Lys Pro Gly Glu
1               5                   10                  15
Ser Leu Lys Ile Ser Cys Lys Ala Ser Gly Tyr Thr Phe Thr Ser Tyr
            20                  25                  30
Val Met His Trp Leu Arg Gln Met Pro Gly Lys Gly Leu Glu Trp Met
        35                  40                  45
Gly Tyr Ile Asn Pro Tyr Asn Asp Gly Thr Lys Tyr Asn Glu Lys Phe
    50                  55                  60
Lys Gly Gln Val Thr Ile Ser Ser Asp Lys Ser Ile Ser Thr Ala Tyr
65                  70                  75                  80
Leu Gln Trp Ser Ser Leu Lys Ala Ser Asp Thr Ala Met Tyr Tyr Cys
                85                  90                  95
Ala Asn Tyr Tyr Gly Ser Lys Phe Ala Met Asp Tyr Trp Gly Gln Gly
            100                 105                 110
Thr Met Val Thr Val Ser Ser
        115

<210>  8
<211>  107
<212>  PRT
<213>  Artificial Sequence

<400>  8
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Asp Ile Ser Asn Tyr
            20                  25                  30
Leu Ser Trp Tyr Gln Gln Lys Pro Gly Gly Ala Val Lys Leu Leu Ile
        35                  40                  45
Tyr Tyr Thr Ser Arg Leu His Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Phe Cys Gln Gln Gly Ile Thr Leu Pro Trp
                85                  90                  95
Thr Phe Gly Gln Gly Thr Lys Val Glu Ile Lys
            100                 105

<210>  9
<211>  165
<212>  PRT
<213>  Homo sapiens

<400>  9

<210> 10
<211> 629
<212> PRT
<213> Artificial Sequence

<400> 10
Glu Val Gln Leu Val Gln Ser Gly Ala Glu Val Lys Lys Pro Gly Glu
1               5                   10                  15
Ser Leu Lys Ile Ser Cys Lys Ala Ser Gly Tyr Thr Phe Thr Ser Tyr
            20                  25                  30
Val Met His Trp Leu Arg Gln Met Pro Gly Lys Gly Leu Glu Trp Met
        35                  40                  45
Gly Tyr Ile Asn Pro Tyr Asn Asp Gly Thr Lys Tyr Asn Glu Lys Phe
    50                  55                  60
Lys Gly Gln Val Thr Ile Ser Ser Asp Lys Ser Ile Ser Thr Ala Tyr
65                  70                  75                  80
Leu Gln Trp Ser Ser Leu Lys Ala Ser Asp Thr Ala Met Tyr Tyr Cys
                85                  90                  95
Ala Asn Tyr Tyr Gly Ser Lys Phe Ala Met Asp Tyr Trp Gly Gln Gly
            100                 105                 110
Thr Met Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys

                        325                             330                             335
Thr Ile Ser Lys Ala Lys Gly Gln Pro Arg Glu Pro Gln Val Tyr Thr
                340                             345                             350
Leu Pro Pro Ser Arg Asp Glu Leu Thr Lys Asn Gln Val Ser Leu Thr
        355                             360                             365
Cys Leu Val Lys Gly Phe Tyr Pro Ser Asp Ile Ala Val Glu Trp Glu
    370                             375                             380
Ser Asn Gly Gln Pro Glu Asn Asn Tyr Lys Thr Thr Pro Pro Val Leu
385                             390                             395                             400
Asp Ser Asp Gly Ser Phe Phe Leu Tyr Ser Lys Leu Thr Val Asp Lys
                        405                             410                             415
Ser Arg Trp Gln Gln Gly Asn Val Phe Ser Cys Ser Val Met His Glu
                420                             425                             430
Ala Leu His Asn His Tyr Thr Gln Lys Ser Leu Ser Leu Ser Pro Gly
        435                             440                             445
Lys Gly Gly Gly Gly Ser Gly Gly Gly Gly Ser Gly Gly Gly Gly Ser
    450                             455                             460
Cys Asp Leu Pro Gln Thr His Ser Leu Gly Ser Arg Arg Thr Leu Met
465                             470                             475                             480
Leu Leu Ala Gln Met Arg Arg Ile Ser Leu Phe Ser Cys Leu Lys Asp
                        485                             490                             495
Arg His Asp Phe Gly Phe Pro Gln Glu Glu Phe Gly Asn Gln Phe Gln
                500                             505                             510
Lys Ala Glu Thr Ile Pro Val Leu His Glu Met Ile Gln Gln Ile Phe
        515                             520                             525
Asn Leu Phe Ser Thr Lys Asp Ser Ser Ala Ala Trp Asp Glu Thr Leu
    530                             535                             540
Leu Asp Lys Phe Tyr Thr Glu Leu Tyr Gln Gln Leu Asn Asp Leu Glu
545                             550                             555                             560
Ala Cys Val Ile Gln Gly Val Gly Val Thr Glu Thr Pro Leu Met Lys
                        565                             570                             575
Glu Asp Ser Ile Leu Ala Val Arg Lys Tyr Phe Gln Arg Ile Thr Leu
                580                             585                             590
Tyr Leu Lys Glu Lys Lys Tyr Ser Pro Cys Ala Trp Glu Val Val Arg
        595                             600                             605
Ala Glu Ile Met Arg Ser Phe Ser Leu Ser Thr Asn Leu Gln Glu Ser
    610                             615                             620
Leu Arg Ser Lys Glu
625

<210>  11
<211>  214
<212>  PRT
<213>  Artificial Sequence

<400>  11
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Asp Ile Ser Asn Tyr
                20                  25                  30
Leu Ser Trp Tyr Gln Gln Lys Pro Gly Gly Ala Val Lys Leu Leu Ile
        35                  40                  45
Tyr Tyr Thr Ser Arg Leu His Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Phe Cys Gln Gln Gly Ile Thr Leu Pro Trp
                85                  90                  95
Thr Phe Gly Gln Gly Thr Lys Val Glu Ile Lys Arg Thr Val Ala Ala
                100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala

130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  12
<211>  445
<212>  PRT
<213>  Artificial Sequence

<400>  12
Glu Val Gln Leu Val Gln Ser Gly Ala Glu Val Lys Lys Pro Gly Glu
1               5                   10                  15
Ser Leu Lys Ile Ser Cys Lys Ala Ser Gly Tyr Thr Phe Thr Ser Tyr
            20                  25                  30
Val Met His Trp Leu Arg Gln Met Pro Gly Lys Gly Leu Glu Trp Met
        35                  40                  45
Gly Tyr Ile Asn Pro Tyr Asn Asp Gly Thr Lys Tyr Asn Glu Lys Phe
    50                  55                  60
Lys Gly Gln Val Thr Ile Ser Ser Asp Lys Ser Ile Ser Thr Ala Tyr
65                  70                  75                  80
Leu Gln Trp Ser Ser Leu Lys Ala Ser Asp Thr Ala Met Tyr Tyr Cys
                85                  90                  95
Ala Asn Tyr Tyr Gly Ser Lys Phe Ala Met Asp Tyr Trp Gly Gln Gly
            100                 105                 110
Thr Met Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Asn Phe Gly Thr Gln Thr Tyr Thr Cys Asn Val Asp His Lys Pro
        195                 200                 205
Ser Asn Thr Lys Val Asp Lys Thr Val Glu Arg Lys Cys Cys Val Glu
    210                 215                 220
Cys Pro Pro Cys Pro Ala Pro Pro Val Ala Gly Pro Ser Val Phe Leu
225                 230                 235                 240
Phe Pro Pro Lys Pro Lys Asp Thr Leu Met Ile Ser Arg Thr Pro Glu
                245                 250                 255
Val Thr Cys Val Val Val Asp Val Ser His Glu Asp Pro Glu Val Gln
            260                 265                 270
Phe Asn Trp Tyr Val Asp Gly Val Glu Val His Asn Ala Lys Thr Lys
        275                 280                 285
Pro Arg Glu Glu Gln Phe Asn Ser Thr Phe Arg Val Val Ser Val Leu
    290                 295                 300
Thr Val Val His Gln Asp Trp Leu Asn Gly Lys Glu Tyr Lys Cys Lys
305                 310                 315                 320
Val Ser Asn Lys Gly Leu Pro Ala Pro Ile Glu Lys Thr Ile Ser Lys
                325                 330                 335
Thr Lys Gly Gln Pro Arg Glu Pro Gln Val Tyr Thr Leu Pro Pro Ser
            340                 345                 350
Arg Glu Glu Met Thr Lys Asn Gln Val Ser Leu Thr Cys Leu Val Lys

355                 360                 365
Gly Phe Tyr Pro Ser Asp Ile Ala Val Glu Trp Glu Ser Asn Gly Gln
    370                 375                 380
Pro Glu Asn Asn Tyr Lys Thr Thr Pro Pro Met Leu Asp Ser Asp Gly
385                 390                 395                 400
Ser Phe Phe Leu Tyr Ser Lys Leu Thr Val Asp Lys Ser Arg Trp Gln
                405                 410                 415
Gln Gly Asn Val Phe Ser Cys Ser Val Met His Glu Ala Leu His Asn
            420                 425                 430
His Tyr Thr Gln Lys Ser Leu Ser Leu Ser Pro Gly Lys
        435                 440                 445

<210>  13
<211>  394
<212>  PRT
<213>  Artificial Sequence

<400>  13
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Asp Ile Ser Asn Tyr
            20                  25                  30
Leu Ser Trp Tyr Gln Gln Lys Pro Gly Gly Ala Val Lys Leu Leu Ile
        35                  40                  45
Tyr Tyr Thr Ser Arg Leu His Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Phe Cys Gln Gln Gly Ile Thr Leu Pro Trp
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys Gly Gly Gly Gly Ser Gly Gly Gly Gly Ser
    210                 215                 220
Gly Gly Gly Gly Ser Cys Asp Leu Pro Gln Thr His Ser Leu Gly Ser
225                 230                 235                 240
Arg Arg Thr Leu Met Leu Leu Ala Gln Met Arg Arg Ile Ser Leu Phe
                245                 250                 255
Ser Cys Leu Lys Asp Arg His Asp Phe Gly Phe Pro Gln Glu Glu Phe
            260                 265                 270
Gly Asn Gln Phe Gln Lys Ala Glu Thr Ile Pro Val Leu His Glu Met
        275                 280                 285
Ile Gln Gln Ile Phe Asn Leu Phe Ser Thr Lys Asp Ser Ser Ala Ala
    290                 295                 300
Trp Asp Glu Thr Leu Leu Asp Lys Phe Tyr Thr Glu Leu Tyr Gln Gln
305                 310                 315                 320
Leu Asn Asp Leu Glu Ala Cys Val Ile Gln Gly Val Gly Val Thr Glu
                325                 330                 335
Thr Pro Leu Met Lys Glu Asp Ser Ile Leu Ala Val Arg Lys Tyr Phe
            340                 345                 350
Gln Arg Ile Thr Leu Tyr Leu Lys Glu Lys Lys Tyr Ser Pro Cys Ala

355                 360                 365
Trp Glu Val Val Arg Ala Glu Ile Met Arg Ser Phe Ser Leu Ser Thr
    370                 375                 380
Asn Leu Gln Glu Ser Leu Arg Ser Lys Glu
385                 390

<210>  14
<211>  449
<212>  PRT
<213>  Artificial Sequence

<400>  14
Glu Val Gln Leu Val Gln Ser Gly Ala Glu Val Lys Lys Pro Gly Glu
1               5                   10                  15
Ser Leu Lys Ile Ser Cys Lys Ala Ser Gly Tyr Thr Phe Thr Ser Tyr
            20                  25                  30
Val Met His Trp Leu Arg Gln Met Pro Gly Lys Gly Leu Glu Trp Met
        35                  40                  45
Gly Tyr Ile Asn Pro Tyr Asn Asp Gly Thr Lys Tyr Asn Glu Lys Phe
    50                  55                  60
Lys Gly Gln Val Thr Ile Ser Ser Asp Lys Ser Ile Ser Thr Ala Tyr
65                  70                  75                  80
Leu Gln Trp Ser Ser Leu Lys Ala Ser Asp Thr Ala Met Tyr Tyr Cys
                85                  90                  95
Ala Asn Tyr Tyr Gly Ser Lys Phe Ala Met Asp Tyr Trp Gly Gln Gly
            100                 105                 110
Thr Met Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys

                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys

<210> 15
<211> 446
<212> PRT
<213> Artificial Sequence

<400> 15
Glu Val Gln Leu Val Gln Ser Gly Ala Glu Val Lys Lys Pro Gly Glu
1               5                   10                  15
Ser Leu Lys Ile Ser Cys Lys Ala Ser Gly Tyr Thr Phe Thr Ser Tyr
            20                  25                  30
Val Met His Trp Leu Arg Gln Met Pro Gly Lys Gly Leu Glu Trp Met
        35                  40                  45
Gly Tyr Ile Asn Pro Tyr Asn Asp Gly Thr Lys Tyr Asn Glu Lys Phe
    50                  55                  60
Lys Gly Gln Val Thr Ile Ser Ser Asp Lys Ser Ile Ser Thr Ala Tyr
65                  70                  75                  80
Leu Gln Trp Ser Ser Leu Lys Ala Ser Asp Thr Ala Met Tyr Tyr Cys
                85                  90                  95
Ala Asn Tyr Tyr Gly Ser Lys Phe Ala Met Asp Tyr Trp Gly Gln Gly
            100                 105                 110
Thr Met Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Phe Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp

385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Lys
        435                 440                 445

<210>  16
<211>  384
<212>  PRT
<213>  Artificial Sequence

<400>  16
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Asp Ile Ser Asn Tyr
            20                  25                  30
Leu Ser Trp Tyr Gln Gln Lys Pro Gly Gly Ala Val Lys Leu Leu Ile
        35                  40                  45
Tyr Tyr Thr Ser Arg Leu His Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Phe Cys Gln Gln Gly Ile Thr Leu Pro Trp
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys Gly Gly Gly Gly Ser Cys Asp Leu Pro Gln
    210                 215                 220
Thr His Ser Leu Gly Ser Arg Arg Thr Leu Met Leu Leu Ala Gln Met
225                 230                 235                 240
Arg Lys Ile Ser Leu Phe Ser Cys Leu Lys Asp Arg His Asp Phe Gly
                245                 250                 255
Phe Pro Gln Glu Glu Phe Gly Asn Gln Phe Gln Lys Ala Glu Thr Ile
            260                 265                 270
Pro Val Leu His Glu Met Ile Gln Gln Ile Phe Asn Leu Phe Ser Thr
        275                 280                 285
Lys Asp Ser Ser Ala Ala Trp Asp Glu Thr Leu Leu Asp Lys Phe Tyr
    290                 295                 300
Thr Glu Leu Tyr Gln Gln Leu Asn Asp Leu Glu Ala Cys Val Ile Gln
305                 310                 315                 320
Gly Val Gly Val Thr Glu Thr Pro Leu Met Lys Glu Asp Ser Ile Leu
                325                 330                 335
Ala Val Arg Lys Tyr Phe Gln Arg Ile Thr Leu Tyr Leu Lys Glu Lys
            340                 345                 350
Lys Tyr Ser Pro Cys Ala Trp Glu Val Val Arg Ala Glu Ile Met Arg
        355                 360                 365
Ser Phe Ser Leu Ser Thr Asn Leu Gln Glu Ser Leu Arg Ser Lys Glu
    370                 375                 380

<210>  17
<211>  385
<212>  PRT
<213>  Artificial Sequence

<400>  17
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Asp Ile Ser Asn Tyr
            20                  25                  30
Leu Ser Trp Tyr Gln Gln Lys Pro Gly Gly Ala Val Lys Leu Leu Ile
        35                  40                  45
Tyr Tyr Thr Ser Arg Leu His Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Phe Cys Gln Gln Gly Ile Thr Leu Pro Trp
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys Gly Gly Gly Gly Ser Met Ser Tyr Asn Leu
    210                 215                 220
Leu Gly Phe Leu Gln Arg Ser Ser Asn Phe Gln Cys Gln Lys Leu Leu
225                 230                 235                 240
Trp Gln Leu Asn Gly Arg Leu Glu Tyr Cys Leu Lys Asp Arg Met Asn
                245                 250                 255
Phe Asp Ile Pro Glu Glu Ile Lys Gln Leu Gln Gln Phe Gln Lys Glu
            260                 265                 270
Asp Ala Ala Leu Thr Ile Tyr Glu Met Leu Gln Asn Ile Phe Ala Ile
        275                 280                 285
Phe Arg Gln Asp Ser Ser Ser Thr Gly Trp Asn Glu Thr Ile Val Glu
    290                 295                 300
Asn Leu Leu Ala Asn Val Tyr His Gln Ile Asn His Leu Lys Thr Val
305                 310                 315                 320
Leu Glu Glu Lys Leu Glu Lys Glu Asp Phe Thr Arg Gly Lys Leu Met
                325                 330                 335
Ser Ser Leu His Leu Lys Arg Tyr Tyr Gly Arg Ile Leu His Tyr Leu
            340                 345                 350
Lys Ala Lys Glu Tyr Ser His Cys Ala Trp Thr Ile Val Arg Val Glu
        355                 360                 365
Ile Leu Arg Asn Phe Tyr Phe Ile Asn Arg Leu Thr Gly Tyr Leu Arg
    370                 375                 380
Asn
385

<210>  18
<211>  362
<212>  PRT
<213>  Artificial Sequence

<400>  18

Asp Ile Gln Met Thr Gln Ser Pro Ser Ser Val Ser Ala Ser Val Gly
1               5                   10                  15
Asp Arg Val Thr Ile Thr Cys Arg Ala Ser Gln Asp Ile Ser Asn Tyr
            20                  25                  30
Leu Ser Trp Tyr Gln Gln Lys Pro Gly Gly Ala Val Lys Leu Leu Ile
        35                  40                  45
Tyr Tyr Thr Ser Arg Leu His Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Phe Cys Gln Gln Gly Ile Thr Leu Pro Trp
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys Gly Gly Gly Gly Ser Gln Asp Pro Tyr Val
    210                 215                 220
Lys Glu Ala Glu Asn Leu Lys Lys Tyr Phe Asn Ala Gly His Ser Asp
225                 230                 235                 240
Val Ala Asp Asn Gly Thr Leu Phe Leu Gly Ile Leu Lys Asn Trp Lys
                245                 250                 255
Glu Glu Ser Asp Arg Lys Ile Met Gln Ser Gln Ile Val Ser Phe Tyr
            260                 265                 270
Phe Lys Leu Phe Lys Asn Phe Lys Asp Asp Gln Ser Ile Gln Lys Ser
        275                 280                 285
Val Glu Thr Ile Lys Glu Asp Met Asn Val Lys Phe Phe Asn Ser Asn
    290                 295                 300
Lys Lys Lys Arg Asp Asp Phe Glu Lys Leu Thr Asn Tyr Ser Val Thr
305                 310                 315                 320
Asp Leu Asn Val Gln Arg Lys Ala Ile His Glu Leu Ile Gln Val Met
                325                 330                 335
Ala Glu Leu Ser Pro Ala Ala Lys Thr Gly Lys Arg Lys Arg Ser Gln
            340                 345                 350
Met Leu Phe Arg Gly Arg Arg Ala Ser Gln
        355                 360

<210>  19
<211>  394
<212>  PRT
<213>  Artificial Sequence

<400>  19
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Asp Ile Ser Asn Tyr
            20                  25                  30
Leu Ser Trp Tyr Gln Gln Lys Pro Gly Gly Ala Val Lys Leu Leu Ile
        35                  40                  45
Tyr Tyr Thr Ser Arg Leu His Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80

Glu Asp Phe Ala Thr Tyr Phe Cys Gln Gln Gly Ile Thr Leu Pro Trp
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys Gly Gly Gly Gly Ser Val Pro Val Ala Arg
    210                 215                 220
Leu Arg Gly Ala Leu Pro Asp Ala Arg Gly Cys His Ile Ala Gln Phe
225                 230                 235                 240
Lys Ser Leu Ser Pro Gln Glu Leu Gln Ala Phe Lys Arg Ala Lys Asp
                245                 250                 255
Ala Leu Glu Glu Ser Leu Leu Leu Lys Asp Cys Lys Cys Arg Ser Arg
            260                 265                 270
Leu Phe Pro Arg Thr Trp Asp Leu Arg Gln Leu Gln Val Arg Glu Arg
        275                 280                 285
Pro Val Ala Leu Glu Ala Glu Leu Ala Leu Thr Leu Lys Val Leu Glu
    290                 295                 300
Ala Thr Ala Asp Thr Asp Pro Ala Leu Gly Asp Val Leu Asp Gln Pro
305                 310                 315                 320
Leu His Thr Leu His His Ile Leu Ser Gln Leu Arg Ala Cys Ile Gln
                325                 330                 335
Pro Gln Pro Thr Ala Gly Pro Arg Thr Arg Gly Arg Leu His His Trp
            340                 345                 350
Leu His Arg Leu Gln Glu Ala Pro Lys Lys Glu Ser Pro Gly Cys Leu
        355                 360                 365
Glu Ala Ser Val Thr Phe Asn Leu Phe Arg Leu Leu Thr Arg Asp Leu
    370                 375                 380
Asn Cys Val Ala Ser Gly Asp Leu Cys Val
385                 390